# EUROPÄISCHE PATENTSCHRIFT

(11) **EP 2 513 063 B1**
(45) Veröffentlichungstag und Bekanntmachung des Hinweises auf die Patenterteilung: **06.11.2013**
(21) Anmeldenummer: 10787791.2
(22) Anmeldetag: 10.12.2010
(51) Int. Cl.: C07D 231/14, C07D 317/30

(54) **VERFAHREN ZUR HERSTELLUNG VON 1-ALKYL-/1-ARYL-5-PYRAZOLCARBONSÄUREDERIVATEN**
Method for manufacturing 1-alkyl-/1-aryl-5-pyrazolcarbonic acid derivatives
Procédé de fabrication de dérivés d'acide carbonique de 1-alkyl-/1-aryl-5-pyrazol

(30) Priorität: 15.12.2009 EP 09179299
(43) Veröffentlichungstag der Anmeldung: 24.10.2012
(73) Patentinhaber: Bayer CropScience AG, 40789 Monheim (DE)
(72) Erfinder: PAZENOK, Sergii, 42699 Solingen (DE); LUI, Norbert, 51519 Odenthal (DE); GERUS, Igor, 02094 Kiew (UA)
(86) Internationale Anmeldenummer: PCT/EP2010/069385
(87) Internationale Veröffentlichungsnummer: WO 2011/073101

(56) Entgegenhaltungen:
- WO-A-2005/049578
- WO-A-2007/144100

## Beschreibung

Die vorliegende Erfindung betrifft ein Verfahren zur Herstellung von 1-Alkyl- oder 1-Aryl-substituierten 5-Pyrazolcarbonsäurederivaten umfassend die Umsetzung von substituierten 1,3-Dioxolanen und 1,4-Dioxanen mit Alkyl- oder Arylhydrazinen zu 1-Alkyl- oder 1-Aryl-substituierten dihydro-1H-Pyrazolen, deren Weiterreaktion unter Wasserabspaltung zu 1-Alkyl- oder 1-Aryl-substituierten Pyrazolen und deren Weiterverarbeitung zu 5-Pyrazolcarbonsäurederivaten.

1-Alkyl-/1-Aryl-substituierte Pyrazole und 1-H-Pyrazole sind wertvolle Zwischenprodukte zur Herstellung von Anthranilsäureamiden, die als Insektizide Verwendung finden können.

In der Literatur ist bereits beschrieben, dass Pyrazole durch Reaktion von 1,3 Dicarbonylen oder entsprechenden 1,3 bis-elektrophilen Reagenzien mit Monoalkyl- oder Monoarylhydrazinen gebildet werden können (Synthesis 2004, N1. pp 43-52). Jedoch wird berichtet, dass im Fall von Monoalkyl- oder Monoarylhydrazinen eine Mischung aus regioisomeren Pyrazolen resultiert (Tetrahedron 59 (2003), 2197-2205; Martins et al., T. L. 45 (2004) 4935). Versuche, exklusiv ein Regioisomer zu erhalten, schlugen fehl (JOC 2007, 72, 8243-8250). In der Literatur ebenfalls beschrieben ist ein Verfahren zur Herstellung von Trifluormethyl-Pyrazolen (WO 2003/016282). Ebenfalls ist Herstellverfahren von (Het)Aryl-substituierten Pyrazolen beschrieben (WO 2007/144100), wobei durch Reduktion von Diestern mit DIBAL oder LiALH₄ die entsprechenden Pyrazole erhalten werden. Allerdings sind dabei sehr tiefe Temperaturen erforderlich, und die Verwendung von DIBAL ist unwirtschaftlich. WO 2010/112178 beschreibt die Herstellung von 5-Pyrazolcarbonsäurederivaten durch Zyklisierung von Acetylenketonen, wobei für die Synthese von Acetylenketonen BuLi und sehr tiefe Temperaturen erforderlich (-70°C bis -80°C) sind.

Die Aufgabe der vorliegenden Erfindung ist daher die Bereitstellung neuer, wirtschaftlicher Verfahren zur Herstellung von 1-Alkyl-/1-Aryl-substituierten 5-Pyrazolcarbonsäurederivaten, die im Pyrazolring in der 3-Position einen weiteren Substituenten (CH₂-R²) tragen. Das Verfahren soll die zuvor beschriebenen Nachteile nicht aufweisen und soll sich durch eine auch im großtechnischen Maßstab besonders gut und einfach durchzuführende Prozeßführung auszeichnen.

Die Aufgabe wurde gemäß der vorliegenden Erfindung gelöst durch ein Verfahren zur Herstellung von 1-Alkyl- /1-Aryl- substituierten 5-Pyrazolcarbonsäurederivaten der allgemeinen Formel (I) 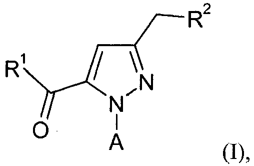 in welcher
- R¹: für Hydroxy, Halogen, Alkoxy, Aryloxy steht,
- R¹: bevorzugt für Hydroxy, Halogen, (C₁-C₆)Alkoxy steht,
- R¹: besonders bevorzugt für Hydroxy, Halogen, (C₁-C₄)Alkoxy steht,
- R²: für Hydroxy, Alkoxy, Arylalkoxy, Halogen, O-(C=O)Alkyl, O-(C=O)O-Alkyl, O(C=O)Halogenalkyl, OSO₂Alkyl, OSO₂ Halogenalkyl, OSO₂-Aryl steht,
- R²: bevorzugt für Hydroxy, Halogen, O-(C=O) (C₁-C₆)Alkyl, OSO₂(C₁-C₆)Alkyl, OSO₂Halogen(C₁-C₆)Alkyl steht,
- R²: besonders bevorzugt für Hydroxy, Halogen, O-(C=O)CH₃ steht,
- A: für Alkyl oder
für die Gruppe 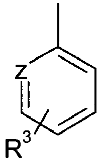 steht,
- A: bevorzugt für (C₁-C₄)Alkyl oder
für die Gruppe 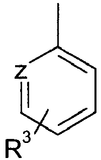 steht,
- A: besonders bevorzugt für die Gruppe 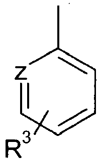 steht,
- R³: für Halogen, CN, NO₂, Alkyl, Cycloalkyl, Halogenalkyl, Halogencycloalkyl, Alkoxy, Halogenalkoxy, Alkylamino, Dialkylamino, Cycloalkylamino steht,
- R³: bevorzugt für Halogen, CN, NO₂, (C₁-C₆)-Alkyl, Halogen(C₁-C₆)-alkyl, (C₁-C₆)Alkoxy, Halogen(C₁-C₆)alkoxy, steht,
- R³: besonders bevorzugt für F, Chlor, Brom, Jod, CN, (C₁-C₄)-Alkyl, Halogen(C₁-C₄)-alkyl, oder Halogen(C₁-C₄)alkoxy steht,
- R³: ganz besonders bevorzugt für Fluor, Chlor, Brom oder Jod steht,
- R³: insbesondere bevorzugt für Chlor steht,
- Z: für CH, N steht,
- Z: bevorzugt und besonders bevorzugt für N steht.
dadurch gekennzeichnet, dass man substituierte 1,3-Dioxolane und 1,4-Dioxane der Formel (II) 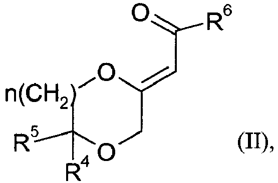 in welcher
- R⁴, R⁵: unabhängig von einander für Wasserstoff, Alkyl, Aryl, Arylalkyl, Alkoxy stehen,
- R⁴, R⁵: weiterhin einen 4, 5, 6 oder 7-gliedrigen, gesättigten, gegebenenfalls substituierten Ring ausbilden können, welcher 1-2 Heteroatome aus der Reihe N,S,O enthalten kann,
- R⁶: für Trihalogenmethyl, (C=O)OAlkyl, (C=O)OHalogenalkyl steht,
- n: für 0 oder 1 steht,
- n: bevorzugt und besonders bevorzugt für 0 steht,
mit Alkyl- oder Arylhydrazinen der Formel (III)

^{A}NHNH₂ (III),
in welcher A für Alkyl oder für die Gruppe 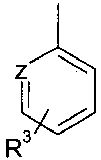 steht,
- R³: für Halogen, CN, NO₂, Alkyl, Cycloalkyl, Halogenalkyl, Halogencycloalkyl, Alkoxy, Halogenalkoxy, Alkylamino, Dialkylamino, Cycloalkylamino steht,
- Z: für CH, N steht,
umsetzt zu 1-Alkyl-/1-Aryl-substituierten dihydro-1H-Pyrazolen der Formel (IV), 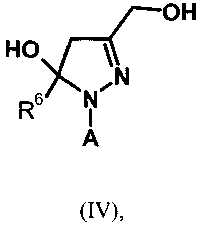 in welcher R⁶, A die oben angegebenen Bedeutungen haben,
diese gegebenenfalls ohne vorherige Isolierung unter Wasserabspaltung weiter umsetzt zu 1-Alkyl-/1-Aryl-substituierten -Pyrazolen der Formel (V) 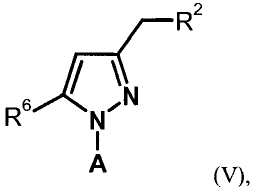 in welcher R², R⁶ und A die oben angegebenen Bedeutungen haben,
diese Verbindungen der allgemeinen Formel (V)
zu Pyrazolcarbonsäurederivaten der Formel (I) umsetzt, 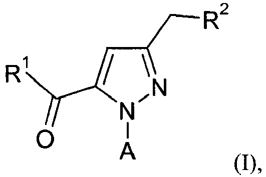 in welcher R¹, R² und A die oben angegebenen Bedeutungen haben.

Insbesondere zeichnet sich das erfindungsgemäße Verfahren durch einen sehr kurzen Syntheseweg, hohe Regioselektivität bei der Bildung des Pyrazolrings, günstige Rohstoffe, wie beispielsweise 2,2-Dimethyl-4-methylene-1,3-dioxolane, 4-Methylene-1,3-dioxolane, Säurechloride und Alkyl- oder Arylhydrazine, sowie durch eine auch im großtechnischen Maßstab besonders gut und einfach durchzuführende Prozeßführung aus.

Das erfindungsgemäße Verfahren kann anhand des folgenden Schemas (I) erläutert werden :

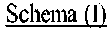

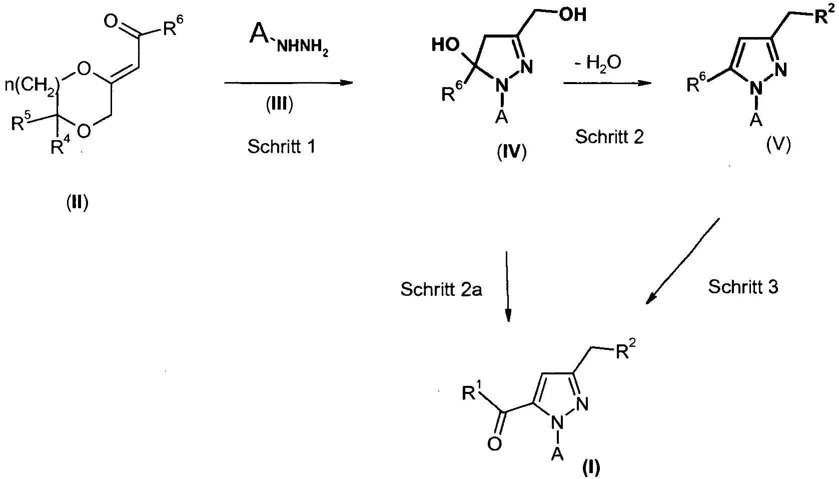

Wobei R¹, R², R⁴, R⁵, R⁶, A, n die oben angegebenen Bedeutungen haben.

Die Verbindungen der Formel (IV), in welcher R⁶ für (C=O)OAlkyl steht, können weiterhin im Schritt (2a) direkt zu Verbindungen der Formel (I) umgesetzt werden, in welcher R¹ für OAlkyl und R² für Hydroxy, Halogen, O-(C=O)(C₁-C₆)Alkyl, OSO₂(C₁-C₆)Alkyl, OSO₂Halogen(C₁-C₆)Alkyl steht.

In einer weiteren Ausführungsform des Verfahrens werden Verbindungen der Formel (II) 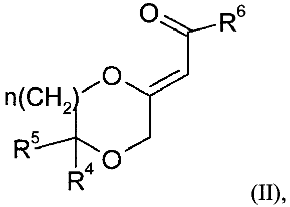 in welcher n für 0 steht und R⁴, R⁵ und R⁶ die oben angegebenen Bedeutungen haben,
zuerst mit Nukleophilen der Formel (VI)

H₂L (VI),

in welcher
- L: für O, NH oder NR⁷ steht,
- R⁷: für Alkyl steht

zu Aminohydroxyoxopentenoaten oder Hydroxy-2,4-dioxopentanoaten der Formel (VII) umgesetzt, welche in Form zweier tautomerer Formen (VIIa) und (VIIb) vorliegen können und einen Ring der Formel (VIIc), (VIId) ausbilden können, 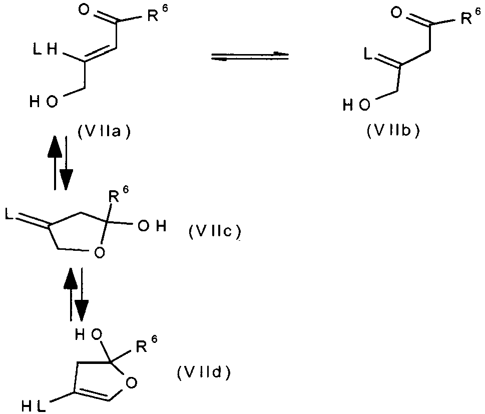 und diese anschließend mit Arylhydrazinen der Formel (III)

A-NHNH₂ (III),

in welcher A die oben angegebenen Bedeutungen hat,
umsetzt zu 1-Alkyl-/1-Aryl-substituierten dihydro-1H-Pyrazolen der Formel (IV), 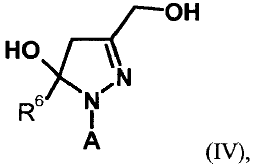 in welcher A und R⁶ die oben angegebenen Bedeutungen haben. Diese können wie oben angegeben zu Verbindungen der allgemeinen Formel (I) weiter umgesetzt werden.

Diese Ausführungsform des erfmdungsgemäßen Verfahrens kann anhand des folgenden Schemas (IA) erläutert werden 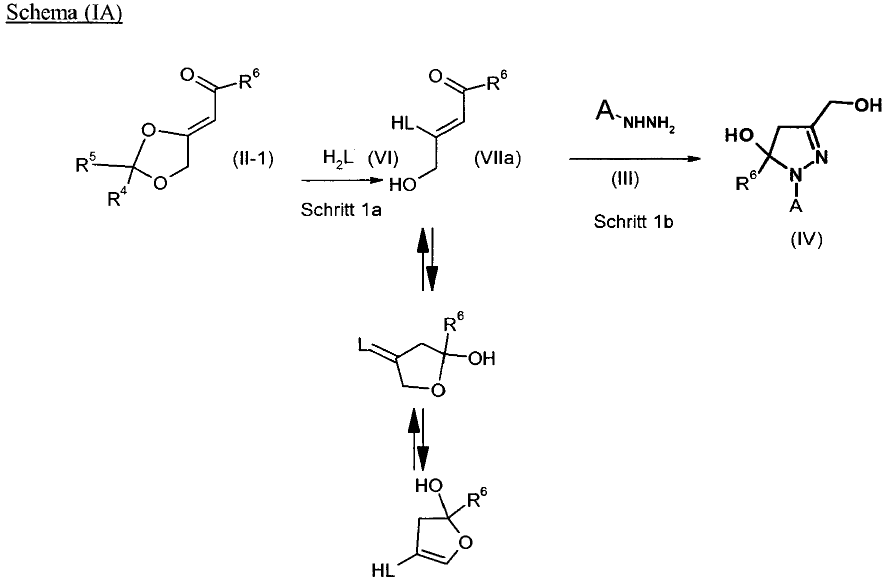 Wobei
die Verbindungen der Formel (II-1) substituierte 1,3 Dioxolane der allgemeinen Formel (II) sind, in welcher n für 0 steht und
R⁴, R⁵, R⁶, A und L die oben angegebenen allgemeinen Bedeutungen haben

### Allgemeine Definitionen:

Im Zusammenhang mit der vorliegenden Erfindung umfasst der Begriff Halogene (X), soweit nicht anders definiert, solche Elemente, die ausgewählt sind aus der Gruppe bestehend aus Fluor, Chlor, Brom und Jod, wobei Fluor, Chlor und Brom bevorzugt und Fluor und Chlor besonders bevorzugt verwendet werden. Substituierte Gruppen können einfach oder mehrfach substituiert sein, wobei bei Mehrfachsubstitutionen die Substituenten gleich oder verschieden sein können.

Mit einem oder mehreren Halogenatomen (-X) substituierte Alkyl-Gruppen = (Halogenalkyl-Gruppen) sind beispielsweise ausgewählt aus Trifluormethyl (CF₃), Difluormethyl (CHF₂), CCl₃, CFCl₂, CF₃CH₂, ClCH₂, CF₃CCl₂.

Alkyl-Gruppen sind im Zusammenhang mit der vorliegenden Erfindung, soweit nicht abweichend definiert, lineare oder verzweigte Kohlenwasserstoff-Gruppen.

Die Definition Alkyl und C₁-C₁₂-Alkyl umfasst beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Cycloalkyl-Gruppen sind im Zusammenhang mit der vorliegenden Erfindung, soweit nicht abweichend definiert, ringförmige gesättigte Kohlenwasserstoff-Gruppen.

Aryl-Reste sind im Zusammenhang mit der vorliegenden Erfindung, soweit nicht abweichend definiert, aromatische Kohlenwasserstoff-Reste, die ein, zwei oder mehrere Heteroatome, die ausgewählt sind aus O, N, P und S aufweisen können und optional durch weitere Gruppen substituiert sein können.

Arylalkyl-Gruppen und Arylalkoxy-Gruppen sind im Zusammenhang mit der vorliegenden Erfindung, soweit nicht abweichend definiert, durch Aryl-Gruppen substituierte Alkyl- bzw. AlkoxyGruppen, die eine Alkylenkette aufweisen können. Im Einzelnen umfasst die Definition Arylalkyl beispielsweise die Bedeutungen Benzyl- und Phenylethyl-; die Definition Arylalkoxy bespielsweise die Bedeutung Benzyloxy.

Alkylaryl-Gruppen (Alkaryl-Gruppen) und Alkylaryloxy-Gruppen sind im Zusammenhang mit der vorliegenden Erfindung, soweit nicht abweichend definiert, durch Alkyl-Gruppen substituierte Aryl-Gruppen, bzw Aryloxy-Gruppen, die eine C₁₋₈-Alkylenkette aufweisen können und im Arylgerüst oder Aryloxygerüst ein oder mehrere Heteroatome, die ausgewählt sind aus O, N, P und S, aufweisen können.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen offenbart und beansprucht.

### Substituierte 1,3 -Dioxolane und 1,4 -Dioxane der Formel (II)

Die bei der Durchführung des erfindungsgemäßen Verfahrens als Ausgangsstoffe verwendeten substituierten 1,3 Dioxolane und 1,4 -Dioxane sind durch die Formel (II) allgemein definiert. 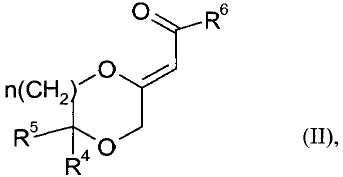 wobei,
- R⁴ und R⁵: unabhängig voneinander für Wasserstoff, Alkyl, Arylalkyl, Aryl oder Alkoxy stehen,
- R⁴, R⁵: weiterhin einen 4, 5 oder 6 gliedrigen, gesättigten, gegebenenfalls substituierten Ring ausbilden können, welcher 1-2 Heteroatome aus der Reihe N,S,O enthalten kann,
- R⁴ und R⁵: unabhängig voneinander bevorzugt für Wasserstoff oder (C₁-C₁₂)Alkyl stehen,
- R⁴ und R⁵: unabhängig voneinander besonders bevorzugt für Wasserstoff oder Methyl stehen;
- n: für 0 oder 1 steht,
- n: bevorzugt und besonders bevorzugt für 0 steht.
- R⁶: für Trihalogenmetlryl, (C=O)OAlkyl, (C=O)OHalogenalkyl steht,
- R⁶: bevorzugt für Trichlormethyl, (C=O)O(C₁-C₆)Alkyl steht,
- R⁶: besonders bevorzugt für Trichlormethyl, (C=O)OMethyl und (C=O)OEthyl steht.
Beispiele für erfindungsgemäß geeignete Dioxalanderivate der Formel (II) sind

1,1,1-Trichlor-3-(2,2-dimethyl-1,3-dioxolan-4-yliden)aceton, 1,1,1-Trifluor-3-(2,2-dimethyl-1,3-dioxolan-4-yliden)aceton, Methyl-3-(2,2-dimethyl-1,3-dioxolan-4-yliden)-2-oxopropanoat, Methyl-3-(1,3-dioxolan-4-yliden)-2-oxopropanoat, Ethyl -3-(2,2-dimethyl-1,3-dioxolan-4-yliden)-2-oxopropanoat oder Ethyl 3-(5,5-dimethyl-1,4-dioxan-2-yliden)-2-oxopropanoat.

Die Verbindungen der Formel (II) sind neu und können hergestellt werden, indem man Verbindungen der allgemeinen Formel (II-a), 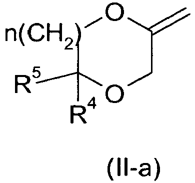 in welcher R⁴, R⁵ die oben angegebenen Bedeutungen haben, mit Anhydriden oder Säurechloriden der allgemeinen Formel (II-b) 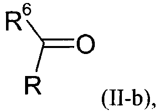 in welcher R für Halogen oder -O(C=O)R⁶ steht und R⁶ die oben angegebenen Bedeutungen hat,
in Gegenwart einer Base umsetzt (vgl. Schema (II)). 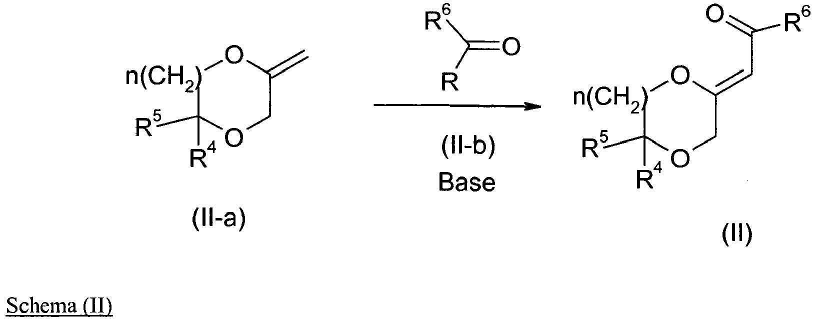 wobei
R⁴, R⁵ und R⁶ die oben angegebenen Bedeutungen haben und R für Halogen oder -O(C=O)R⁶ steht.

### Alkyl- und Arylhydrazine der allgemeinen Formel (III)

Die gemäß der vorliegenden Erfindung verwendeten Alkyl- oder Arylhydrazine sind Verbindungen der allgemeinen Formel (III)

^{A}NHNH₂ (III),

in welcher
- A: für Alkyl oder
für die Gruppe 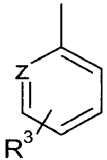 steht,
- A: bevorzugt für (C₁-C₄)Alkyl oder
für die Gruppe 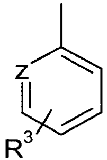 steht,
- A: besonders bevorzugt für die Gruppe 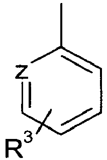 steht,
- R³: für Halogen, CN, NO₂, Alkyl, Cycloalkyl, Halogenalkyl, Halogencycloalkyl, Alkoxy, Halogenalkoxy, Alkylamino, Dialkylamino, Cycloalkylamino, steht,
- R³: bevorzugt für Halogen, CN, NO₂, (C₁-C₆)-Alkyl, Halogen(C₁-C₆)-alkyl, (C₁-C₆)Alkoxy, Halogen(C₁-C₆)alkoxy steht,
- R³: besonders bevorzugt für F, Chlor, Brom, Jod, CN, (C₁-C₄)-Alkyl, Halogen(C₁-C₄)-alkyl oder Halogen(C₁-C₄)alkoxy steht,
- R³: ganz besonders bevorzugt für Fluor, Chlor, Brom oder Jod steht,
- R³: insbesondere bevorzugt für Chlor steht,
- Z: für CH, N steht,
- Z: bevorzugt und besonders bevorzugt für N steht.

Beispiele für ein erfindungsgemäß geeignete Hydrazine sind Methylhydrazin, Ethylhydrazin, 3-Chloro-2-hydrazinopyridin, Phenylhydrazin, o- und p-Chlorphenylhydrazin, o- und p-Methylphenylhydrazin, Nitrophenylhydrazine. Diese Verbindungen sind kommerziell erhältlich.

### Schritt (1)

In einer ersten Ausführungsform des vorliegenden Verfahrens, werden zunächst substituierte 1,3-Dioxolane oder 1,4-Dioxane der Formel (II) mit Alkyl- oder Arylhydrazinen der Formel (III) umgesetzt. 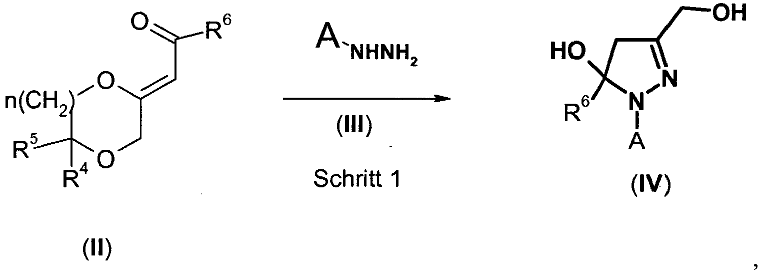 in welcher R⁴, R⁵ , R⁶ und A die oben angegebenen Bedeutungen haben.

Überraschenderweise wurde gefunden, dass die Umsetzung von substituierten 1,3-Dioxolanen oder 1,4-Dioxanen der Formel (II) mit Alkyl- oder Arylhydrazinen der Formel (III) selektiv erfolgt zu 1-Alkyl-/1-Aryl-substituierten dihydro-1H-Pyrazolen der Formel (IV). Das zweite mögliche Regioisomer wurde nicht beobachtet. Auch ist es als überraschend anzusehen, dass am Ende der Reaktion mit Dioxolanen der Formel (II-1) ein geringer Teil (ca unter 3 %) des Hydrazins der allgemeinen Formel (III) durch Reaktion mit in der Reaktion abgespaltenem Keton der allgemeinen Formel (VIII) in Hydrazon der allgemeinen Formel (IX) umgewandelt wurde. Überraschenderweise reagiert das Hydrazon der allgemeinen Formel (IX) mit Dioxolanen der Formel (II-1) zur Verbindung der Formel (IV).

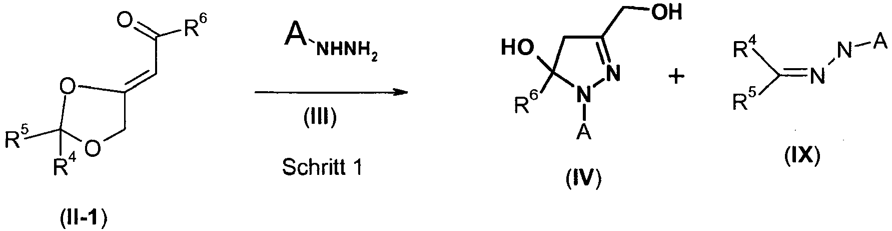

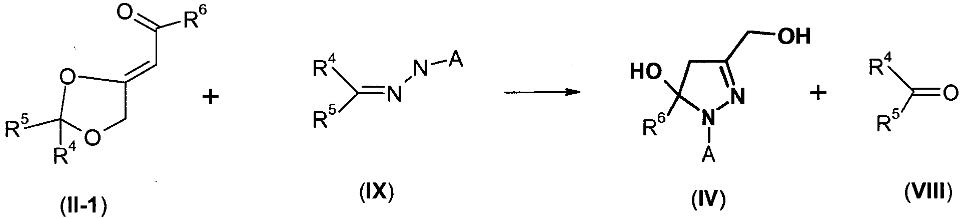

Die Durchführung des erfindungsgemäßen Verfahrensschritts (1) erfolgt vorzugsweise innerhalb eines Temperaturbereichs von -20°C bis +100°C, besonders bevorzugt bei Temperaturen von -10°C bis +80 °C., besonders bevorzugt 20 bis 60°C.

Der erfindungsgemäße Verfahrensschritt (1) wird im Allgemeinen unter Normaldruck durchgeführt. Alternativ ist es jedoch auch möglich, im Vakuum zu arbeiten, um im Verfahren gebildetes Keton aus dem Reaktionsgemisch zu entfernen.

Die Reaktionszeit ist nicht kritisch und kann, in Abhängigkeit vom Substrat, von der Ansatzgröße und Temperatur, in einem Bereich zwischen wenigen und mehreren Stunden gewählt werden.

Bei der Durchführung des erfindungsgemäßen Verfahrensschritts setzt man 1 Mol des substituierten 1,3-Dioxolanes oder 1,4-Dioxanes der Formel (II) mit 0,8 Mol bis 2 Mol, vorzugsweise 0,9 Mol bis 1,7 Mol, besonders bevorzugt mit 1,0-1,2 Mol. des Alkyl- oder Arylhydrazins der Formel (III) um.

Geeignete Lösungsmittel sind beispielsweise aliphatische, alicyclische oder aromatische Kohlenwasserstoffe, wie z.B. Petrolether, n-Hexan, n-Heptan, Cyclohexan, Methylcyclohexan, Benzol, Toluol, Xylol oder Decalin, und halogenierte Kohlenwasserstoffe, wie z.B. Chlorbenzol, Dichlorbenzol, Dichlormethan, Chloroform, Tetrachlormethan, Dichlorethan oder Trichlorethan, Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Nitrile, wie Acetonitril, Propionitril, n- oder iso-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid oder Sulfone, wie Sulfolan, Alkohole wie Methanol, Ethanol, Isopropanol. Besonders bevorzugt verwendet man Toluol, Ethanol, Methyltert.Butylether, THF, Isopropanol, Acetonitril.

### Schritt (1a)

In einer weiteren Ausführungsform des vorliegenden Verfahrens, werden zunächst substituierte 1,3-Dioxolane der Formel (II-1) mit Nukleophilen der Formel (VI) umgesetzt (vgl. Schema (IA)). Dadurch wird Keton der allgemeinen Formel (VIII) freigesetzt und vor der Durchführung des Schritts 1b entfernt.

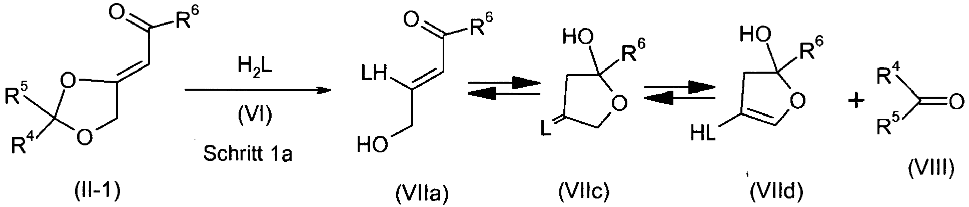

Die Verbindungen der allgemeinen Formel (VII) sind neu.

Sie können in verschiedenen tautomeren Formen vorliegen, z.B als Hydroxyacetonderivate oder einen Ring ausbilden, z.B. als zyklisches 2-Hydroxy-4-oxotetrahydrofuran. Die Verbindungen der allgemeinen Formel (VII) zeigen bei der Durchführung dieser Ausführungsform des erfindungsgemäßen Verfahrens die gleiche Reaktivität. In Abhängigkeit von der Polarität und Acidität des Lösungsmittels und der Temperatur liegen unterschiedliche Formen der Verbindungen der allgemeinen Formel (VII) vor.

Die Durchführung des erfindungsgemäßen Verfahrensschritts (1a) erfolgt vorzugsweise innerhalb eines Temperaturbereichs von -20°C bis +100°C, besonders bevorzugt bei Temperaturen von -10°C bis +80 °C, besonders bevorzugt bei +20°C bis 60°C. So reagieren Dioxolane der allgemeinen Formel (II-1) mit Ammoniak schon bei 0°C binnen weiniger Minuten zum Alkyl- 4-amino-5-hydroxy-2-oxopent-3-enoate.Für die Reaktion von Dioxolane der Formel (II-1) mit Wasser benötigt man dagegen mehrere Stunden bei Raumtemperatur.

Der erfindungsgemäße Verfahrensschritt (1a) wird im Allgemeinen unter Normaldruck durchgeführt. Besonders vorteilhaft ist es, im Vakuum zu arbeiten, wobei das gebildete Keton der allgemeinen Formel (VIII) aus dem Gemisch entfernt wird.

Die Reaktionszeit ist nicht kritisch und kann, in Abhängigkeit von Substrat, Ansatzgröße und Temperatur, in einem Bereich zwischen wenigen Minuten und mehreren Stunden gewählt werden.

Bei der Durchführung des erfindungsgemäßen Verfahrensschritts (1 a) setzt man 1 Mol des substituierten 1,3-Dioxolanes der Formel (II) mit 0,8 Mol bis 2 Mol, vorzugsweise 0,9 Mol bis 1,7 Mol, besonders bevorzugt mit 1-1,3 Mol. der Nukleophile der Formel VI um. Es ist möglich die Reaktion in Wasser durchzuführen wobei das Wasser als Reagenz und Lösemittel dient.

Die Isolierung der Verbindungen der Formel VII a erfolgt durch Filtration z.B für Feststoffe wie

Methyl- 4-amino-5-hydroxy-2-oxopent-3-enoate oder durch Extraktion im Falle von flüssigen Intermediaten.

Es ist auch möglich, die Verbindungen ohne Isolierung weiter umzusetzen.

Geeignete Lösungsmittel sind beispielsweise aliphatische, alicyclische oder aromatische Kohlenwasserstoffe, wie z.B. Petrolether, n-Hexan, n-Heptan, Cyclohexan, Methylcyclohexan, Benzol, Toluol, Xylol oder Decalin, und halogenierte Kohlenwasserstoffe, wie z.B. Chlorbenzol, Dichlorbenzol, Dichlormethan, Chloroform, Tetrachlormethan, Dichlorethan oder Trichlorethan, Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Nitrile, wie Acetonitril, Propionitril, n- oder iso-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid oder Sulfone, wie Sulfolan, Alkohole wie Methanol, Ethanol, Isopropanol, Wasser. Besonders bevorzugt verwendet man Acetonitrile, Isopropanol, Wasser

### Schritt 1b

In dieser Ausführungsform des erfindungsgemäßen Verfahrens werden die im Schritt 1a gebildeten Verbindungen der Formel (VII) mit Alkyl- oder Arylhydrazinen der Formel (III) umgesetzt. 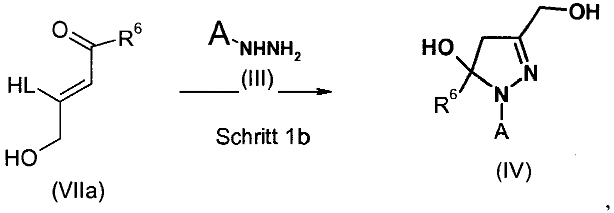 in welcher R⁴ , R⁵, R⁶, A und L die oben angegebenen Bedeutungen haben.

Überraschenderweise wurde gefunden, dass die Umsetzung von Aminoalkoholen oder Alkyl 5-hydroxy-2,4-dioxopentanoate der allgemeinen Formel (VII) mit Alkyl- oder Arylhydrazinen der Formel (III) selektiv erfolgt zu 1-Alkyl-/1-Aryl-substituierten dihydro-1H-Pyrazolen der Formel (IV).

Die Durchführung des erfindungsgemäßen Verfahrensschritts (1b) erfolgt vorzugsweise innerhalb eines Temperaturbereichs von -20°C bis +100°C, bevorzugt bei Temperaturen von -10°C bis +80 °C, besonders bevorzugt bei Temperaturen von +20°C bis +60°C.

Der erfindungsgemäße Verfahrensschritt (1b) wird im Allgemeinen unter Normaldruck durchgeführt. Alternativ ist es jedoch auch möglich, im Vakuum zu arbeiten,

Die Reaktionszeit ist nicht kritisch und kann, in Abhängigkeit von der Ansatzgröße und Temperatur, in einem Bereich zwischen wenigen und mehreren Stunden gewählt werden.

Bei der Durchführung des erfindungsgemäßen Verfahrensschritts (1b) setzt man 1 Mol der Verbindung der Formel (VII) mit 0,8 Mol bis 2 Mol, vorzugsweise 0,9 Mol bis 1,7 Mol, besonders bevorzugt mit 1 bis 1,3 Mol. des Alkyl- oder Arylhydrazins der Formel (III) um.

Die Reaktion kann durch Zusatz von Säuren beschleunigt werden. Geeignete Säuren sind HCl, H₂SO₄, CF₃COOH, Trifluormethansulfonsäure, p-Toluolsulfonsäure, Methansulfonsäure.

Man setzt die Säure in Mengen von 0,2 bis 2 Mol, bevorzugt 0,5 bis 1,1 Mol bezogen auf die Verbindung der Formel (VII) ein.

Geeignete Lösungsmittel sind beispielsweise aliphatische, alicyclische oder aromatische Kohlenwasserstoffe, wie z.B. Petrolether, n-Hexan, n-Heptan, Cyclohexan, Methylcyclohexan, Benzol, Toluol, Xylol oder Decalin, und halogenierte Kohlenwasserstoffe, wie z.B. Chlorbenzol, Dichlorbenzol, Dichlormethan, Chloroform, Tetrachlormethan, Dichlorethan oder Trichlorethan, Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Nitrile, wie Acetonitril, Propionitril, n- oder iso-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid oder Sulfone, wie Sulfolan, Alkohole wie Methanol, Ethanol, Isopropanol. Besonders bevorzugt verwendet man Toluol, Ethanol, Methyltert.Butylether, THF, Isopropanol, Acetonitril.

Die gebildeten 1-Alkyl-/1-Aryl-substituierten dihydro-1H-Pyrazole der Formel (IV) können ohne vorherige Aufarbeitung im darauffolgenden Schritt (2 oder 2a), in welchem Wasserabspaltung stattfindet, eingesetzt werden.

Alternativ können die Verbindungen der Formel (IV) durch geeignete Aufarbeitungsschritte und ggf. weitere Aufreinigung isoliert werden. Erst zu einem späteren Zeitpunkt kann dann Wasser abgespalten werden.

### Schritte 2 und 2a. Aromatisierung durch Wasserabspaltung

In einer bevorzugten Ausführungsform des erfindungsgemäßen Verfahrens werden die im Schritt 1 gebildeten Verbindungen der Formel (IV) zu 1-Alkyl- oder 1-Aryl-substituierten Pyrazolen der Formel (V) unter Wasserabspaltung umgesetzt (vgl. Schritt 2 im Schema (I)). 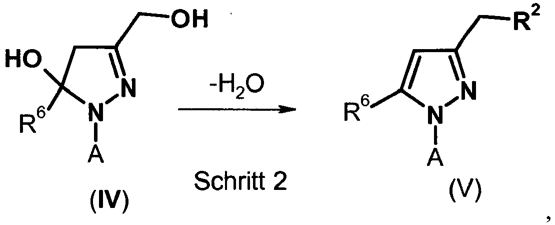 in welcher A, R², R⁶ die oben angegebenen Bedeutungen haben.

Für die Wasserabspaltung kommen folgende Reagenzien in Frage: HCl, H₂SO₄, CF₃COOH, Trifluormethansulfonsäure, Pivaloylchlorid, PCl₅, POCl₃, P₄O₁₀, Polyphosphorsäure, SOCl₂, (CH₃CO)₂O, (CF₃CO)₂O, Oxalylchlorid, Methansulfonsäure, p-Toluolsulfonsäure, Phosgen und Diphosgen, Methansulfonsäurechlorid (MesCl), SiO₂.

Bevorzugt sind HCl, (CF₃CO)₂O, MesCl, Thionylchlorid, Acetanhydrid, Oxalylchlorid, Phosgen und P₄O₁₀.

Während der Wasserabspaltung mit Anhydriden und Halogenanhydriden (z.B SOCl₂, POCl₃, Oxalylchlorid, Phosgen, MesCl), findet auch die Derivatisierung der CH₂OH-Gruppe statt, sodass man in nur einem Schritt die Verbindungen der Formel (V), in welcher R² für Chlor, Brom, Fluor, Jod, O-(C=O)Alkyl, O-(C=O)O-Alkyl, O(C=O)Halogenalkyl, OSO₂Alkyl, OSO₂-Halogenalkyl oder OSO₂-Aryl steht, erhält.

Die Umsetzung mit Säuren wie HCl, H₂SO₄, H₃PO₄, Polyphosphorsäure liefert die Verbindungen der Formel (V), in welchen R² für OH steht. Falls R⁶ für (C=O)OAlkyl steht, ist es vorteilhaft, mit HCl in Methanol zu arbeiten, um das Produkt der Formel (V), wobei R⁶ für (C=O)OAlkyl und R² für OH steht , in hoher Ausbeute zu erhalten.

Es ist auch möglich, Wasser durch thermische Belastung (Erhitzen) abzuspalten.

Die Durchführung des erfindungsgemäßen Verfahrensschritts (2) erfolgt vorzugsweise innerhalb eines Temperaturbereichs von -20°C bis +180°C, besonders bevorzugt bei Temperaturen von -10°C bis +150 °C.

Der erfindungsgemäße Verfahrensschritt (2) wird im Allgemeinen unter Normaldruck durchgeführt. Alternativ ist es jedoch auch möglich, im Vakuum oder unter Überdrück (z.B. Umsetzung mit Phosgen) zu arbeiten.

Die Reaktionszeit ist nicht kritisch und kann, in Abhängigkeit von der Ansatzgröße und Temperatur, in einem Bereich zwischen wenigen Minuten und mehreren Stunden gewählt werden.

Bei der Durchführung des erfindungsgemäßen Verfahrensschritts setzt man 1 Mol der Verbindung der Formel (III) mit 0,1 Mol bis 2,5 Mol, vorzugsweise 1 Mol bis 1,8 Mol, besonders bevorzugt mit der äquimolaren Menge des Entwässerungsmittels um.

Falls zusätzlich noch Derivatisierung stattfindet, setzt man 1 Mol der Verbindung der Formel (IV) mit 1 Mol bis 3 Mol, vorzugsweise 1,5 Mol bis 2,5 Mol, besonders bevorzugt mit 1,8 bis 2,5 Mol des Entwässerungsmittels um.

Es ist auch möglich, das Wasser katalytisch abzuspalten (HCl, SiO₂, H₂SO4).

Geeignete Lösungsmittel sind beispielsweise aliphatische, alicyclische oder aromatische Kohlenwasserstoffe, wie z.B. Petrolether, n-Hexan, n-Heptan, Cyclohexan, Methylcyclohexan, Benzol, Toluol, Xylol oder Decalin, und halogenierte Kohlenwasserstoffe, wie z.B. Chlorbenzol, Dichlorbenzol, Dichlormethan, Chloroform, Tetrachlormethan, Dichlorethan oder Trichlorethan, Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Nitrile, wie Acetonitril, Propionitril, n- oder iso-Butyronitril oder Benzonitril; Ketone, wie Aceton, Methylethylketon, Methylisobutylketon oder Cyclohexanon; Amide, wie N,N-Dimethylformamid, N,N-Dimethylacelamid, N-Methylformanilid, N-Methylpyrrolidon oder Hexamethylphosphorsäuretriamid; Sulfoxide, wie Dimethylsulfoxid oder Sulfone, wie Sulfolan. Alkohole wie Methanol, Ethanol, Isopropanol Besonders bevorzugt verwendet man Methanol, Methyl-tert-butylether, Toluol, Xylol, Dichlorethan, Dichlormethan, Chlorbenzol, Cyclohexan oder Methylcyclohexan, ganz besonders bevorzugt Methanol, Toluol, Xylol, THF, CH₂Cl₂, Dichlorethan, Methyl-tert-butylether, Acetonitril. Es ist auch möglich, die Reaktion ohne Lösungsmittel durchzuführen, z.B in Substanz.

Weiterhin kann die Aromatisierung unter basischen Bedingungen durchgeführt werden (vgl. Schritt (2a) in Schema (I)), um die Verbindung der Formel (I), in welcher R¹ und R² für OH stehen, in nur einem Schritt zu erhalten. Dafür geeignet sind Basen wie beispielsweise LiOH, NaOH, KOH oder CsOH. Geeignete Lösungsmittel sind Alkohole oder Wasser. 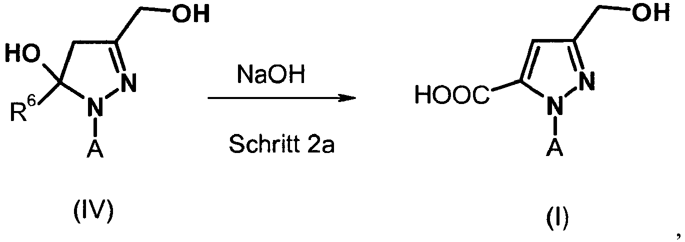 wobei R⁶ für (C=O)OAlkyl steht und A die oben angegebenen Bedeutungen hat.

### Schritt 3

In einer weiteren bevorzugten Ausführungsform des erfindungsgemäßen Verfahrens werden die 1-Alkyl-/1-Aryl-substituierte-Pyrazole der Formel (V) direkt zur Verbindung der Formel (I) umgewandelt (vgl. Schritt 3 in Schema (I)). 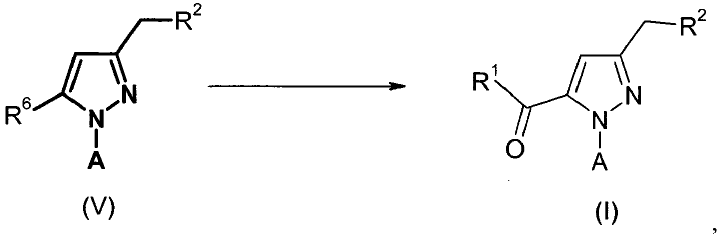 wobei R¹, R², A, R⁶ die oben angegebenen Bedeutungen haben. Dabei werden die Transformationen in der R⁶ und/oder in der R² Gruppe durchgeführt.

Für die Transformation R⁶= Trihalogenmethyl zu R¹=OH wird die Reaktion in der Regel unter sauren oder basischen Bedingungen durchgeführt. Bevorzugt sind mineralische Säuren, beispielsweise H₂SO₄, HCl, HSO₃Cl, HF, HBr, HI, H₃PO₄ oder organische Säuren, beispielsweise CF₃COOH, p-Toluolsulfonsäure, Methansulfonsäure, Trifluormethansulfonsäure. Die Reaktion kann durch der Zusatz von Katalysatoren wie beispielsweise FeCl₃, AlCl₃, BF₃, SbCl₃, NaH₂PO₄ beschleunigt werden. Die Reaktion kann ebenfalls ohne Zusatz von Säure nur in Wasser durchgeführt werden.

Basische Hydrolyse erfolgt in Gegenwart von organischen Basen wie Trialkylamine, Alkylpyridine, Phosphazene und 1,8-Diazabicyclo[5.4.0]undecen (DBU), anorganischen Basen wie, Alkalimetallhydroxide wie z.B. Lithium-, Natrium- oder Kaliumhydroxid, Alkalimetallcarbonate wie beispielsweise Na₂CO₃, K₂CO₃ und -Acetate wie beispielsweise NaOAc, KOAc, LiOAc, sowie - Alkoholate, wie z.B. NaOMe, NaOEt, NaOt-Bu, KOt-Bu.

Die Durchführung des erfindungsgemäßen Verfahrensschritts (3) erfolgt vorzugsweise innerhalb eines Temperaturbereichs von 20°C bis +150°C, besonders bevorzugt bei Temperaturen von 30°C bis +110 °C.

Der erfindungsgemäße Verfahrensschritt (3) wird im Allgemeinen unter Normaldruck durchgeführt. Alternativ ist es jedoch auch möglich, im Vakuum oder unter Überdruck (z.B. Umsetzung im Autoklav mit wässriger HCl, oder mit Methanol) zu arbeiten.

Die Reaktionszeit kann, in Abhängigkeit von der Ansatzgröße und Temperatur, in einem Bereich zwischen 1 Stunde und mehreren Stunden gewählt werden.

Für die Transformation R⁶= Trihalogenmethyl zu R¹=Alkoxy benutzt man z.B. Alkohole beispielsweise Methanol, Ethanol, Propanol oder die Kombinationen Alkohol/HCl, Alkohol/FeCl₃, Alkohol/H₂SO₄ oder Alkohol/Alkoholat.. Dabei dient der Alkohol als Reagenz und als Lösemittel gleichzeitig. Für die Umsetzung beispielsweise mit Methanol oder Ethanol ist es vorteilhaft, die Reaktion unter Druck durchzuführen, um die Reaktionstemperatur von 90° oder von 90°-100°C zu erreichen, und damit die Reaktionszeit zu verkürzen.

Der Reaktionsschritt 3 kann in Substanz oder in einem Lösungsmittel durchgeführt werden. Vorzugsweise wird die Reaktion in einem Lösungsmittel durchgeführt. Geeignete Lösungsmittel sind beispielsweise ausgewählt aus der Gruppe bestehend aus Wasser, aliphatischen und aromatischen Kohlenwasserstoffen, wie z.B. n-Hexan, Benzol oder Toluol, die durch Fluor- und Chloratome substituiert sein können, wie Methylenchlorid, Dichlorethan, Fluorbenzol, Chlorbenzol oder Dichlorbenzol; Ethern, wie z.B. Diethylether, Diphenylether Methyl-tert-butylether, Isopropylethylether, Dioxan, Diglym, Dimethylglycol, Dimethoxyethane (DME) oder THF; Nitrilen wie Methylnitril, Butylnitril oder Phenylnitril; Amide wir Dimethylformamid (DMF) oder N-methlypyrollidon (NMP) oder Mischungen solcher Lösungsmittel geeignet, wobei Wasser, Acetonitril, Dichlormethan und Alkohole besonders gut geeignet sind.

### Herstellbeispiele

Die folgenden Herstellbeispiele illustrieren die Erfindung, ohne sie zu beschränken.

Insbesondere illustrieren die Beispiele 1, 2, 10, 11 die Herstellung von Pyrazolverbindungen der Formel IV (Schritt 1). Die Beispiele 7, 12, 13 illustrieren Schritt 2. Beispiele 3,5,6 illustrieren Schritt 2a. und Beispiel 9 illustriert Schritt 3.

### Beispiele 1

### Methyl 1-(3-chloropyridin-2-yl)-5-hydroxy-3-(hydroxymethyl)-4,5-dihydro-1H-pyrazol-5-carboxylat

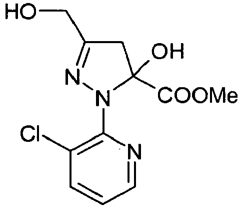

Das Gemisch von Methyl 3-(2,2-dimethyl-1,3-dioxolan-4-yliden)-2-oxopropanoat (20 g., 0.1 mol) und 2-Hydrazino-3-chlorpyridin (14,3 g, 0.1 mol) in 40 Isopropanol wurde 18 Std. bei 35°C gerührt. Der Niederschlag wurde abfiltriert und mit 15 ml Isopropanol gewaschen. Man erhielt 24,2 g (85 %) des Produktes als hell-gelben Feststoff mit einem Schmelzpunkt von 113°C.

### Analytische Charakterisierung

¹H NMR (DMSO d₆) δ: 7.99 (1H, d); 7,65 (1H, d); 6.85 (1H, dd); 6.4 (1H, b.s); 4,51 (2H, b.s); 3,25 (1 H, d); 3,05 (1H,d), 2,55 (s, 1H) ppm.

### Beispiel 2

Ethyl 1-(3-chloropyridin-2-yl)-5-hydroxy-3-(hydroxymethyl)-4,5-dihydro-1H-pyrazol-5-carboxylat

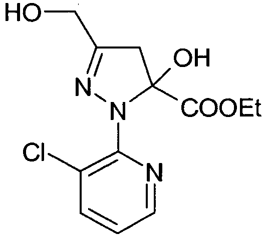

Das Gemisch von Ethyl (3E)-3-(2,2-dimethyl-1,3-dioxolan-4-yliden)-2-oxopropanoat (21.4 g, 0.1 mol) und 2-Hydrazino-3-chlorpyridin (14,3 g , 0.1 mol) in 50 ml Ethanol wurde 18 Std. bei 35°C gerührt. Ethanol wurde im Vakuum entfernt und der Rückstand in 100 ml Methyl(tert)butylether aufgenommen. Die organische Phase wurde 1 mal mit 50 ml 1%iger HCl gewaschen und eingeengt. Man erhielt 26,2 g (86 % Ausbeute) des Produktes als zähflüssiges Öl mit der Reinheit (HPLC) von 97%.

### Analytische Charakterisierung

¹H NMR (DMSO d₆) δ: 7.99 (1H, d); 7,65 (1H, d); 6.85 (1H, dd); 6.0 (OH, b.s); 4,51 (2H, b.s); 4.25 (2H, q); 3,25 (1H, d); 3,05 (1H,d); 1,28 (t, 3H) ppm.

### Beispiel 3

### Methyl 1-(3-chlorpyridin-2-yl)-3-(hydroxymethyl)-1H-pyrazole-5-carboxylat

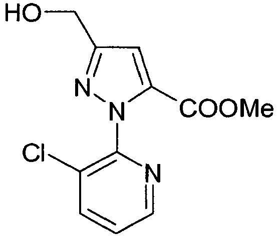

Zu der Suspension von (28,5 g, 0,1 mol) Methyl 1-(3-chloropyridin-2-yl)-5-hydroxy-3-(hydroxymethyl)-4,5-dihydro-1H-pyrazole-5-carboxylate in 100 ml Methanol die Lösung von HCl (9,1 g , 4 % Lösung in Methanol) wurden zugegeben. Nach ca. 30-60 Min bei 25-30°C die klare gelbe Lösung entstanden. Methanol wurde im Vakuum entfernt und der Niederschlag mit Wasser gewaschen. Ausbeute 26,7 g., 100 %. Schmp. 104°C.

### Analytische Charakterisierung

¹H NMR (DMSO d₆) δ: 8,52 (1H,d); 8,06 (1H,d); 7,55 (1H, dd); 7,10 (1H, s); 5,4 (1H, b.s) 4,5 (2H,s); 3,75 (3H,s) ppm.

### Beispiel 4

### Methyl 1-(3-chlorpyridin-2-yl)-3-(hydroxymethyl)-1H-pyrazol-5-carboxylat

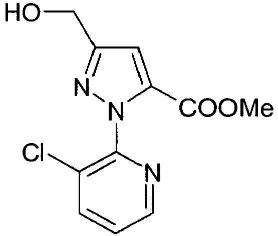

(32,6 g, 0,1 Mol) [1-(3-chlorpyridin-2-yl)-5-(trichlormethyl)-1H-pyrazol-3-yl]methanol
und 300 ml Methanol wurden 3 Std. bei 90°C im Autoklaven erhitzt. Methanol wurde im Vakuum entfernt und der Niederschlag mit Wasser gewaschen. Ausbeute 25 g. 88 %.

Schmp.104°C.

### Beispiel 5

### Methyl 1-(3-chlorpyridin-2-yl)-3-{[(methylsulfonyl)oxy]methyl}-1H-pyrazol-5-carboxylat

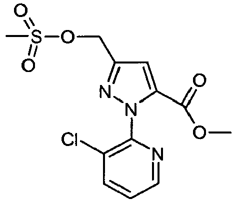

Methyl 1-(3-chlorpyridin-2-yl)-5-hydroxy-3-(hydroxymethyl)-4,5-dihydro-1H-pyrazol-5-carboxylat (28.5 g, 0.1 mol) und 15 g Triethylamin wurden in 150 ml THF vorgelegt und die Lösung auf 5°C gekühlt. (11,4 g, 0,1 Mol) Mesylchlorid wurde bei 0-5°C binnen 20 min zugegeben und das Gemisch bei 0°C 2 Std. nachgerührt. Das Reaktionsgemisch wurde mit Wasser verdünnt und das Produkt mit Ethylacetat extrahiert. Die Ethylacetat-Lösung wurde gewaschen, getrocknet und eingeengt. Der zähölige Rückstand (Auswage 31 g) enthielt nach LC/MS 98 % des Produktes.

### Analytische Charakterisierung

¹H NMR (DMSO d₆) δ: 8,58 (1H,d); 8,27 (1H,d); 7,73 (1H,dd); 7,29 (1H,s); 5,35 (2H,s); 3,75 (3H, q); 3,25 (3H,s) ppm. M/Z 345.

### Beispiel 6

### Methyl 3-(chlormethyl)-1-(3-chlorpyridin-2-yl)-1H-pyrazol-5-carboxylat

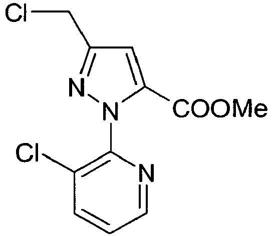

Methy 1-(3-chlorpyridin-2-yl)-5-hydroxy-3-(hydroxymethyl)-4,5-dihydro-1H-pyrazol-5-carboxylat (28.5 g, 0.1 mol) wurde in 100 ml CH₃CN gelöst und die Lösung auf 70°C erhitzt. (26 g , 0.22 mol) SOCl₂ wurde langsam bei dieser Temperatur zugetropft. Das Gemisch wurde 1 Std. bei 70°C nachgerührt und im Vakuum eingeengt. Man erhielt 27,6 (92 %) des Produktes als zähflüssiges braunes Öl mit der Reinheit von 95 %.

### Analytische Charakterisierung

¹H NMR (CD₃CN) δ: 8,52 (1H, d); 8,06 (1H,d); 7,55 (1H, dd); 7,10 (1H, s); 4,75 (2H, s); 3,75 (3H,s) ppm.

### Beispiel 7

### 1-(3-Chlorpyridin-2-yl)-5-(trichlonnethyl)-1H-pyrazol-3-yl]methyl acetat.

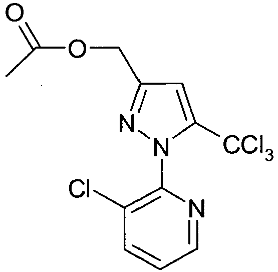

1-(3-Chlorpyridin-2-yl)-3-(hydroxymethyl)-5-(trichlormethyl)-4,5-dihydro-1H-pyrazol-5-ol (34.3 g, 0,1 Mol) und (12,2 g.,0,12 Mol) Acetanhydrid wurden 1 Std. bei 80°C erhitzt und das Reaktionsgemisch im Vakuum 1 mbar komplett eingeengt. Man erhielt 35 g des Produktes als zähes Öl, welches nach ca. 8 Std. bei Raumtemperatur kristallisiert. Schmp. 40°C.

### Analytische Charakterisierung

¹H NMR (DMSO d₆) δ: 8.5 (1H, dd); 8.1 (1H, dd); 7.6 (1H, dd); 7.0 (1H,s) ; 5.1 (2H, dd), 2.0 (3H,s) ppm.

### Beispiel 8

### [1-(3-Chlorpyridin-2-yl)-5-(trichlormethyl)-1H-pyrazol-3-yl]methanol

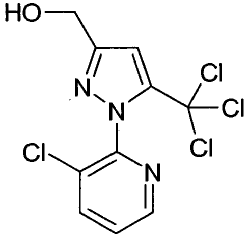

1-(3-Chlorpyridin-2-yl)-5-(trichlormethyl)-1H-pyrazol-3-yl]methyl acetat (36.9 g. 0,1 Mol) wurden in 100 ml Ethanol gelöst und 10 g NaOH (als 40 % Lösung in Wasser) wurden zugegeben. Nach 1 Std. wurde das Gemisch mit 300 ml Wasser verdünnt, das Produkt wurde abfiltriert, mit Wasser gewaschen und getrocknet. Man erhielt 31 g (95 %) des Produktes als weißer Feststoff.

Schmp. 109-111°C.

### Analytische Charakterisierung

¹H NMR (DMSO d₆) δ : 8.5 (1H, dd); 8.05 (1H, dd); 7.55 (1H,dd); 6.95 (1H,s); 5,35 (1H,bs), 4.55 (2H,s) ppm.

### Beispiel 9

### 1-(3-Chlorpyridin-2-yl)-3-(hydroxymethyl)-1H-pyrazol-5-carbonsäure

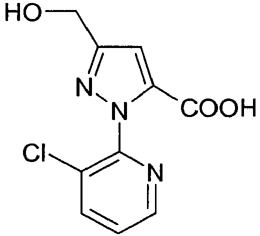

38.7 g (0.1 mol) [1-(3-Chlorpyridin-2-yl)-5-(trichlormethyl)-1H-pyrazol-3-yl]methanol und 10 g H₂SO₄ (als 10 % Lösung in Wasser) wurden 3 Stunden bei 80°C gerührt. Das Gemisch wurde abgekühlt auf 0°C , mit Lösung von NaHCO₃ neutral gestellt und der Niederschlag wurde abfiltriert, mit Acetonitril gewaschen und getrocknet. Ausbeute 90 %. Schmp. 178-180 °C.

### Analytische Charakterisierung

¹H NMR (DMSO d₆) δ: 12,8 (1H, b.s); 8.45 (1H, dd); 8.1 (1H,dd); 7.55 (1H, dd); 6.95 (1H,s);

5.2 (1H, b.s); 4.50 (2H,s) ppm.

### Beispiel 10

### 3-(Hydroxymethyl)-1-phenyl-5-(trifluomethyl)-4,5-dihydro-1H-pyrazol-5-ol

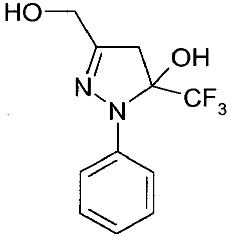

Man arbeitet wie in Beispiel 1 beschrieben, verwendet jedoch Phenylhydrazin und 1,1,1-Trifluor-3-(2,2-dimethyl-1,3-dioxolan-4-yliden)aceton.

Ausbeute (62 %), Schmp. 72-74 °C.

### Analytische Charakterisierung

¹H NMR (DMSO-d₆) δ: 7.98 (1H, b.s); 7.32 (2H, m), 7.24 (2H, m), 6.94 (1H, m), 5.50-5.00 (1H, b.s), 4.20 (2H, s), 3.43 and 3.21 (2H, AB system, *J*_{HH}=19.1Hz, C*H*₂) ppm.

### Beispiel 11

### 3-(Hydroxymethyl)-1-phenyl-5-(trichlormethyl)-4,5-dihydro-1H-pyrazol-5-ol

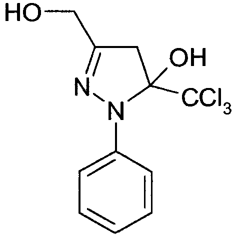

Man arbeitet wie in Beispiel 2 beschrieben, verwendet jedoch Phenylhydrazin und 1,1,1-Trichlor-3-(2,2-dimethyl-1,3-dioxolan-4-yliden)aceton.

### Ausbeute (68%), Schmp. 122-124 °C (Zersetzung).

### Analytische Charakterisierung

¹H NMR ( DMSO-d₆) δ: 8.17 (1H, b.s), 7.49 (2H, m), 7.21 (2H, m), 6.96 (1H, m), 4.70-4.30 (1H, b.s), 4.18 (2H,s), 3.64 and 3.34 (2H, AB system, *J*_{HH}=19.3Hz, C*H*₂) ppm.

### Beispiel 12

### (1-Phenyl-5-(triftuormethyl)-1H-pyrazol-3-yl)methanol

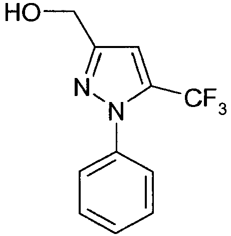

### 3-(Hydroxymethyl)-1-phenyl-5-(trifluormethyl)-4,5-dihydro-1H-pyrazol-5-ol (5 g)

wurde erhitzt bei 100-120°C für 30 min. Das Produkt wurde isoliert und gereinigt mittels Säulenchromatographie (Eluent: Ethylacetat:Hexane 1/1-Mischung), Ausbeute 1,5 g (32%).

### Analytische Charakterisierung

¹H NMR (CDCl₃) δ: 7.46 (5H, m), 6.80 (1H, s), 4.73 (2H, s), 2.50 (1H, br. s);

¹⁹F NMR (CDCl₃) δ: -58.16 (s, C*F*₃) ppm.

### Beispiel 13

### 1-Phenyl-5-(trichlormethyl)-1H-pyrazol-3-yl)methanol.

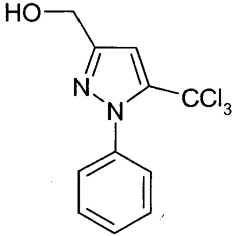

Man arbeitet wie in Beispiel 12 beschrieben, verwendet jedoch 3-(Hydroxymethyl)-1-phenyl-5-(trichlonnethyl)-4,5-dihydro-1H-pyrazol-5-ol.

Ausbeute 21%.

### Analytische Charakterisierung

¹H NMR (CDCl₃) δ: 7.6-7.5 (5H, m), 7.00 (1H, br. s), 4.74 (2H, s), 2.47 (1H, b. s) ppm.

Desweiteren illustrieren insbesondere die Herstellbeispiele, 14, 15, 16 und 17 die Herstellung von Intermediaten der Formel (VII), (Schritt 1a) der weiteren Ausführungsform des erfindungsgemäßen Verfahrens, Beispiele 18, 19, 20 und 21 die Herstellung von Pyrazolverbindungen der Formel (IV) (Schritt 1b).

### Beispiel 14

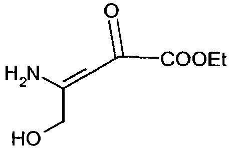

### (Z)- and (E)- Ethyl 4-amino-5-hydroxy-2-oxopent-3-enoate

Zu der Lösung von (6 g, 28 mmol) von (E)-Ethyl 3-(1,3-dioxolan-4-ylidene)-2-oxopropanoate in 25 ml acetonitrile, 2,7 mL (28 mmol) Ammoniak (als 19 % Lösung in Wasser) wurde zugegeben.

Das Reaktiongemisch wurde 3 h bei 25 °C nachgerührt und im Vakuum eingeengt. Der Rückstand wurde mit Hexan gewaschen. 3.8 g (78.4%) wurden erhalten.

¹H NMR (DMSO-d₆) δ: *cis*-isomer (~90%): 9.89 (1H,b.s, N*H*), 8.28 (1H, b.s, 1H, N*H*), 5.66 (1H, s, C*H*), 5.61 (1H, b.s, O*H*), 4.15 (2H, q, OC*H*₂), 4.14 (2H, s, C*H*₂), 1.22 (3H, t, C*H*₃); *trans*-isomer (~10%): 8.37 (1H, b. s, N*H*), 7.11 (1H, b. s, N*H*), 5.75 (1H, s, C*H*), 4.57 (2H,s, C*H*₂).

### Beispiel 15

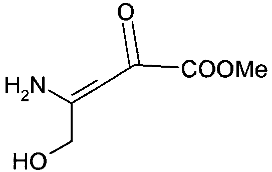

### (Z)- und (E)-Methyl- 4-amino-5-hydroxy-2-oxopent-3-enoate

Zu der Lösung von (9 g, 45 mmol) von Methyl (3E)-3-(2,2,-dimethyl-1,3-dioxolan-4-ylidene)-2-oxopropanote in 50 ml acetonitrile Ammoniak Lösung (2,32 g , 45 mmol, 33 % Lösung in Wasser) wurde zugegeben. Nach ca. 2 Std. der weißer Feststoff wurde abgesaugt und mit kaltem Acetonitril gewaschen. Man erhält 5,36 g (75 %) des Produktes mit Schmp.130-132 °C.

¹H NMR (DMSO-d₆) δ: *cis*-isomer (95 %): 9.91 (1H, b.s, N*H*), 8.35 (1H, b.s, N*H*), 5.68 (1H, s ), 5.67 (1H, b.s, 1H, O*H*), 4.15 (2H, s), 3,7 (3H, s), *trans*-isomer (~5%): 8.36 (1H, b.s, N*H*), 7.12 (1H, b.s, N*H*), 5.76 (1H,s, C*H*), 4,15 (2H, s), 3,68 (3H, s) ppm.

### Beispiel 16

### 5-Hydroxy-5-(trifluoromethyl)dihydrofuran-3(2H)-one

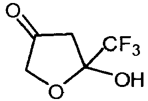

Ein Gemisch aus 3-(2,2-dimethyl-1,3-dioxolan-4-ylidene)-1,1,1-trifluoroacetone (5.21 g, 24.8 mmol) und 20 ml Wasser wurden 24 Std bei 20 °C gerührt. Die flüchtige Komponenten wurden in Vakuum 20 mbar entfernt. Das Produkt wurde mit Dichlormethane extragiert und organische Phase über MgSO₄ getrocknet und eingeengt. Der Niederschlag wurde durch Kristallization aus Toluol gereinigt.

Ausbeute. 3.0 g (71.1%), Schmp 45-47 °C.

¹H NMR (DMSO-d₆) δ: 8.08 (1H,s), 4.28 (2H, m), 3.05 and 2.65 (AB-System, C*H*₂, *J*_{HH} = 18.2 Hz) ppm.

¹⁹F NMR (DMSO-d₆) -85.24 (s) ppm.

### Beispiel 17

### Ethyl 2-hydroxy-4-oxotetrahydrofuran-2-carboxylate

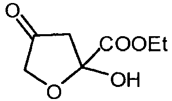

Man arbeitet wie in Beispiel 16 beschrieben, nimmt jedoch Ethyl 3-(2,2,-dimethyl-1,3-dioxolan-4-ylidene)-2-oxopropanote.

Ausbeute 77 %.

¹H NMR (CDCl₃) δ: 4.62 (1H, b.s), 4.28 (2H, q), 4.22 und 4.12 (AB-system, *J*_{HH} = 16.6 Hz); 3.05 and 2.59 (AB-System, *J*_{HH} = 18.3 Hz), 1.30 (3H, t) ppm.

### Beispiel 18

### Herstellung von Methyl 1-(3-chloropyridin-2-yl)-5-hydroxy-3-(hydroxymethyl)-4,5-dihydro-1H-pyrazol-5-carboxylat über Methyl 2-hydroxy-4-oxotetrahydrofuran-2-carboxylate

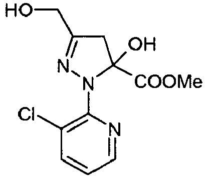

2 g (10 mmol) Methyl-3-(2,2,-dimethyl-1,3-dioxolan-4-ylidene)-2-oxopropanote und 20 ml Waser wurden 18 Std. bei RT gerührt. Der Niederschlag geht in die Lösung. Die Lösung wurde 1 Std in Vakuum 100 mbar gerührt, dabei ca. 10 ml der Flüssigkeit wurden entfernt. 10 ml Isopropanol und (1,43 g. 10 mmol) 3-Chlorpyridin-2-ylhydrazine wurden zugegeben und das Gemisch wurde 24 Std. bei RT nachgerührt. Der Niederschlag wurde abfiltriert und mit Isopropanol gewaschen. Man erhielt 2,1 g (74 %), des Produktes mit Schmp. 111-113°C.

### Beispiel 19

### Ethyl 1-(3-chloropyridin-2-yl)-5-hydroxy-3-(hydroxymethyl)-4,5-dihydro-1H-pyrazole-5-carboxylate

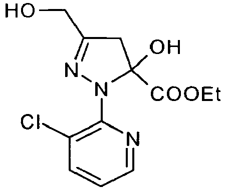

Das Gemisch aus Ethyl 4-amino-5-hydroxy-2-oxopent-3-enoate (2.5 g, 14 mmol), 3-Chlorpyridin-2-ylhydrazine (2.07 g, 14 mmol) und p-Toluosulfonsäure Monohydrat (2.5 g, 13 mmol) wurde in 25 ml Acetonitril 5 Std. bei 25 °C gerührt. Der Niederschlag wurde abfiltriert und der Filtrat wurde in Vakuum eingeengt. Das Produkt wurde durch Säulen-Chromatographie auf SiO₂ gereinigt (Eluent Hexan/Etylacetat). Öl. Ausbeute 4.2 g (90%).

¹H NMR (DMSO-d₆) δ: 8.02 (1H, m), 7.78 (1H, m), 6.88 (1H, m), 5.60 (1H, b. s), 5.29 (1H, b. s), 4.24 (2H, s), 4.13 (2H, q), 3.18 und 2.94 (2H, AB system, *J*_{HH}=17.9Hz,), 1.08 (3H, t) ppm..

### Beispiel 20

**Methyl** 1-(3-chloropyridin-2-yl)-5-hydroxy-3-(hydroxymethyl)-4,5-dihydro-1H-pyrazol-5-carboxylat.

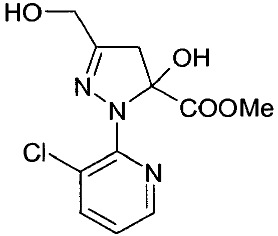

Das Gemisch aus Methyl 4-amino-5-hydroxy-2-oxopent-3-enoate (1,59 g, 10 mmol), 3-Clorpyridin-2-ylhydrazine (1,43 g, 10 mmol) und HCl (1g, 10 mmol, 37 % Lösung in Wasser) in 15 ml Acetonitril wurde 20 Std. bei 25 °C gerührt. Die Lösung wurde in Vakuum eingeengt und der Rückstand mit Wasser und Isopropanol gewaschen. Man erhielt 2,13 g (75 %) des Produktes mit Schmelzpunkt von 111-113°C.

### Analytische Charakterisierung

¹H NMR (DMSO d₆ ) δ: 7.99 (1H, d); 7,65 (1H, d); 6.85 (1R, dd); 6.4 (1H, b.s); 4,51 (2H, b.s); 3,25 (1H, d); 3,05 (1H,d), 2,55 (s, 1H) ppm.

### Beispiel 21

### Ethyl 1-(3-chloropyridin-2-yl)-3-(hydroxymethyl)-1H-pyrazole-5-carboxylate.

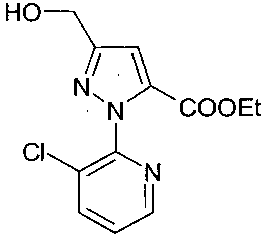

Man arbeitet wie in Beispiel 3 beschrieben, verwendet jedoch Ethyl 1-(3-chloropyridin-2-yl)-5-hydroxy-3-(hydroxymethyl)-4,5-dihydro-1H-pyrazol-5-carboxylat.

Ausbeute 98 %. Zähflüssiges Öl.

¹H NMR (DMSO-d₆) δ: 8.56 (1H, m), 8.24 (1H, m), 7.68 (1H, m), 7.07 (1H, s), 5.39 (1H, b. s), 4.54 (2H, s), 4.14 (2H, q), 1.09 (3H, t) ppm.

## Patentansprüche

1. Verfahren zur Herstellung von 1-Alkyl- /1-Aryl- substituierten 5-Pyrazolcarbonsäurederivaten der allgemeinen Formel (I) 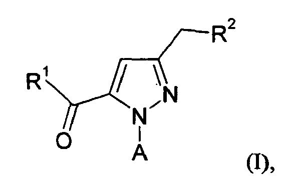 in welcher
R¹ für Hydroxy, Halogen, Alkoxy, Aryloxy steht,
R² für Hydroxy, Alkoxy, Arylalkoxy, Halogen, O-(C=O)Alkyl, O-(C=O)O-Alkyl, O(C=O)Halogenalkyl, OSO₂Alkyl, OSO₂ Halogenalkyl, OSO₂-Aryl steht,
A für Alkyl oder
für die Gruppe 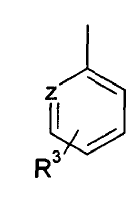 steht,
R³ für Halogen, CN, NO₂, Alkyl, Cycloalkyl, Halogenalkyl, Halogencycloalkyl, Alkoxy, Halogenalkoxy, Alkylamino, Dialkylamino, Cycloalkylamino steht,
Z für CH, N steht,
**dadurch gekennzeichnet, dass** man substituierte 1,3-Dioxolane und 1,4-Dioxane der Formel (II) 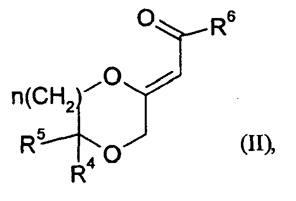 in welcher
R⁴, R⁵ unabhängig von einander für Wasserstoff, Alkyl, Aryl, Arylalkyl, Alkoxy stehen,
R⁴, R⁵ weiterhin einen 4, 5 oder 6 gliedrigen, gesättigten, gegebenenfalls substituierten Ring ausbilden können, welcher 1-2 Heteroatome aus der Reihe N,S,O enthalten kann,
R⁶ für Trihalogenmethyl, (C=O)OAlkyl, (C=O)OHalogenalkyl steht,
n für 0 oder 1 steht,
mit Alkyl- oder Arylhydrazinen der Formel (III)
^{A}NHNH₂ (III),
in welcher A für Alkyl oder für die Gruppe 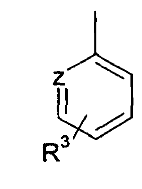 steht,
R³ für Halogen, CN, NO₂, Alkyl, Cycloalkyl, Halogenalkyl, Halogencycloalkyl, Alkoxy, Halogenalkoxy, Alkylamino, Dialkylamino, Cycloalkylamino steht,
Z für CH, N steht,
umsetzt zu 1-Alkyl-/1-Aryl-substituierten dihydro-1H-Pyrazolen der Formel (IV), 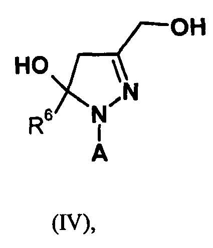 in welcher R⁶, A die oben angegebenen Bedeutungen haben,
diese gegebenenfalls ohne vorherige Isolierung unter Wasserabspaltung weiter umsetzt zu 1-Alkyl-/1-Aryl-substituierten -Pyrazolen der Formel (V) 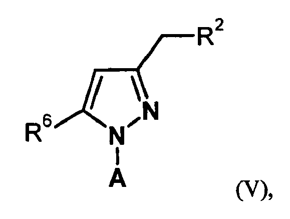 in welcher R², R⁶ und A die oben angegebenen Bedeutungen haben, diese Verbindungen der allgemeinen Formel (V)
zu Pyrazolcarbonsäurederivaten der Formel (I) umsetzt, 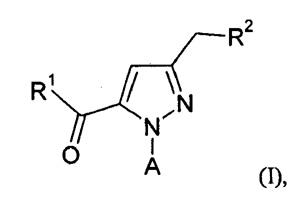 in welcher R¹, R² und A die oben angegebenen Bedeutungen haben.

2. Verfahren zur Herstellung von Verbindungen der Formel (I) gemäß Anspruch 1, wobei
R¹ für Hydroxy, Halogen, (C₁-C₆)Alkoxy steht,
R² für Hydroxy, Halogen, O-(C=O) (C₁-C₆)Alkyl, OSO₂(C₁-C₆)Alkyl, OSO₂-Halogen(C₁-C₆)Alkyl steht,
A für (C₁-C₄)Alkyl oder
für die Gruppe 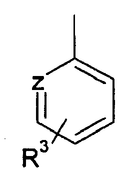 steht,
R³ für Halogen, CN, NO₂, (C₁-C₆)-Alkyl, Halogen(C₁-C₆)-alkyl, (C₁-C₆)Alkoxy, Halogen(C₁-C₆)alkoxy, steht,
Z für N steht.

3. Verfahren zur Herstellung von Verbindungen der Formel (I) gemäß einem der Ansprüche 1 oder 2, wobei
R¹ für Hydroxy, Halogen, (C₁-C₄)Alkoxy steht,
R² für Hydroxy, Halogen, O-(C=O)CH₃ steht,
A für die Gruppe 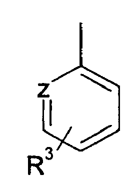 steht,
R³ für Chlor steht,
Z für N steht.

4. Verfahren zur Herstellung von Verbindungen der Formel (I) gemäß einem der Ansprüche 1 bis 3, **dadurch gekennzeichnet, dass** n für 0 steht.

5. Verfahren zur Herstellung von Verbindungen der Formel (I) gemäß einem der Ansprüche 1 bis 4, **dadurch gekennzeichnet, dass** man Verbindungen der Formel (II) 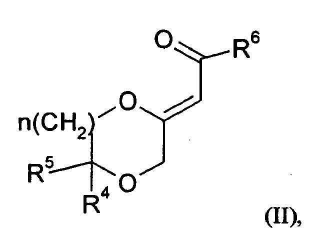 in welcher n für 0 steht und R⁴, R⁵ und R⁶ die oben angegebenen Bedeutungen haben,
zuerst mit Nukleophilen der Formel (VI)
H₂L (VI), in welcher L für O, NH oder NR⁷ steht,
R⁷ für Alkyl steht,
zu Aminohydroxyoxopentenoaten oder Hydroxy-2,4-dioxopentanoaten der Formel (VII) umsetzt, welche in Form zweier tautomerer Formen (VIIa) und (VIIb) vorliegen können und einen Ring der Formel (VIIc), (VIId) ausbilden können, 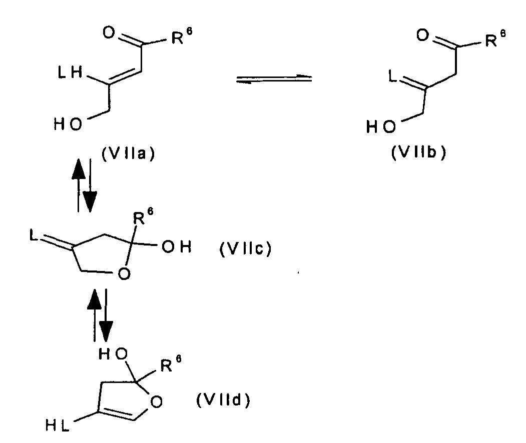 und diese anschließend mit Arylhydrazinen der Formel (III) A-NHNH₂ (III),
in welcher A die oben angegebenen Bedeutungen hat,
umsetzt zu 1-Alkyl-/1-Aryl-substituierten dihydrö-1H-Pyrazolen der Formel (IV), 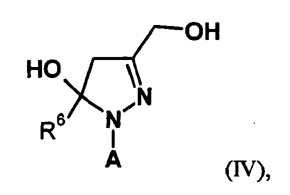 in welcher A und R⁶ die oben angegebenen Bedeutungen haben.

6. Verfahren zur Herstellung von Verbindungen der Formel (I) gemäß einem der Ansprüche 1 bis 6, **dadurch gekennzeichnet, dass** die Durchführung des Verfahrensschritts (1) innerhalb eines Temperaturbereichs von -20°C bis +100°C erfolgt.

7. Verfahren zur Herstellung von Verbindungen der Formel (I) gemäß einem der Ansprüche 1 bis 4, **dadurch gekennzeichnet, dass** 1 Mol einer Verbindung der Formel (II) mit 0,8 Mol bis 2 Mol eines
Alkyl- oder Arylhydrazins der Formel (III) umgesetzt wird.

8. Verfahren zur Herstellung von Verbindungen der Formel (I) gemäß einem der Ansprüche 1 bis 7, **dadurch gekennzeichnet, dass**
die Verbindungen der Formel (IV) unter Zugabe einer Base direkt in die Verbindungen der Formel (I) umgesetzt werden, wobei
R¹ und R² für Hydroxy stehen, R⁶ für (C=O)OAlkyl steht, und
R³,R⁴,R⁵,A und Z die Bedeutung gemäß Anspruch 1 haben.

9. Verfahren zur Herstellung von Verbindungen der Formel (I) gemäß Anspruch 8, **dadurch gekennzeichnet, dass** die Base ausgewählt ist aus der Gruppe bestehend aus LiOH, NaOH, KOH und CsOH und als Lösungsmittel Alkohole oder Wasser verwendet werden.

10. Verfahren zur Herstellung von Verbindungen der Formel (I) gemäß einem der Ansprüche 1 bis 7, **dadurch gekennzeichnet, dass** die Durchführung des Verfahrensschritts (3) innerhalb eines Temperaturbereichs von 20°C bis +150°C erfolgt.

11. Verfahren zur Herstellung von Verbindungen der Formel (I) gemäß einem der Ansprüche 1 bis 7 oder 10, **dadurch gekennzeichnet, dass** die Durchführung des Verfahrensschritts (3) unter sauren Bedingungen mit mineralischen oder organischen Säuren oder unter basischen Bedingungen mit organischen oder anorganischen Basen erfolgt.

12. Verfahren zur Herstellung von Verbindungen der Formel (I) gemäß einem der Ansprüche 1 bis 7 oder 10, **dadurch gekennzeichnet, dass** die Durchführung des Verfahrensschritts (3) mit Alkohol oder Kombinationen von Alkohol/HCl, Alkohol/FeCl₃, Alkohol/H₂SO₄ oder Alkohol/Alkoholat erfolgt.

13. Verfahren zur Herstellung von Verbindungen der Formel (I) gemäß einem der Ansprüche 1 bis 7, **dadurch gekennzeichnet, dass**
die Verbindungen der Formel (IV) unter Zugabe einer Säure (HCl) direkt in die Verbindungen der Formel (I) umgesetzt werden, wobei
R¹ für (C=O)OAlkyl, R² für Hydroxy und R⁶ für (C=O)OAlkyl stehen, und
R³,R⁴,R⁵,A und Z die Bedeutung gemäß Anspruch 1 haben.

14. Verbindungen der Formel (IV) 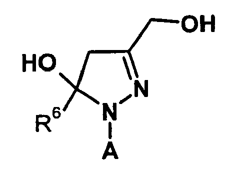 In welcher R⁶ für (C=O)OAlkyl steht und A die in Anspruch 1 angegebene Bedeutung hat.

15. Verbindungen der allgemeinen Formel (VII), welche in Form zweier tautomerer Formen (VIIa) und (VIIb) vorliegen können und einen Ring der Formel (VIIc), (VIId) ausbilden können, 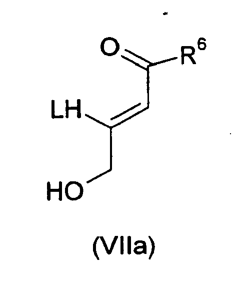 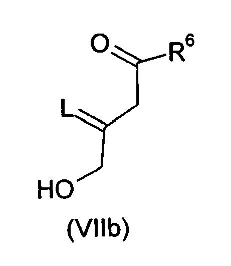 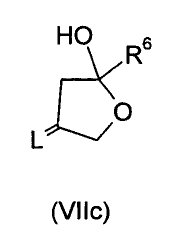 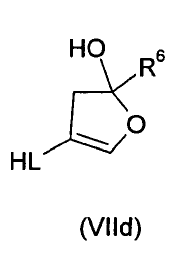 wobei L für O oder NH steht und R⁶ für CF₃ oder (C=O)OAlkyl steht.

## Claims

1. Process for the preparation of 1-alkyl-/1-aryl-substituted 5-pyrazolecarboxylic acid derivatives of the general formula (I) 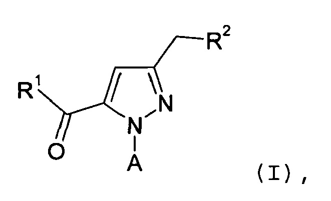 in which
R¹ is hydroxy, halogen, alkoxy, aryloxy,
R² is hydroxy, alkoxy, arylalkoxy, halogen, O-(C=O)alkyl, O-(C=O)O-alkyl, O(C=O)haloalkyl, OSO₂alkyl, OSO₂haloalkyl, OSO₂-aryl,
A is alkyl or
is the group 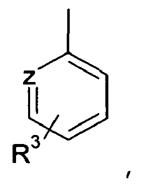
R³ is halogen, CN, NO₂, alkyl, cycloalkyl, haloalkyl, halocycloalkyl, alkoxy, haloalkoxy, alkylamino, dialkylamino, cycloalkylamino,
Z is CH, N,
**characterized in that** substituted 1,3-dioxolanes and 1,4-dioxanes of the formula (II) 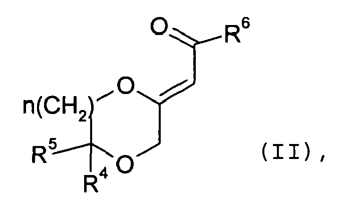 in which
R⁴, R⁵ independently of one another are hydrogen, alkyl, aryl, arylalkyl, alkoxy,
R⁴, R⁵ can furthermore form a 4-, 5- or 6-membered, saturated, optionally substituted ring which can contain 1-2 heteroatoms from the series N, S, O,
R⁶ is trihalomethyl, (C=O)Oalkyl, (C=O)Ohaloalkyl,
n is 0 or 1,
with alkyl- or arylhydrazines of the formula (III)
^{A}NHNH₂ (III),
in which A is alkyl or is the group 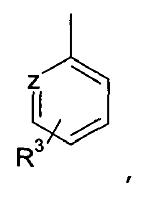
R³ is halogen, CN, NO₂, alkyl, cycloalkyl, haloalkyl, halocycloalkyl, alkoxy, haloalkoxy, alkylamino, dialkylamino, cycloalkylamino,
Z is CH, N,
are converted to 1-alkyl-/1-aryl-substituted dihydro-1H-pyrazoles of the formula (IV), 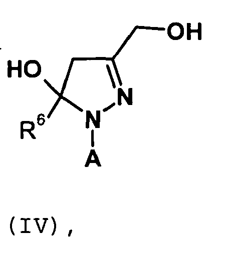 in which R⁶, A have the meanings given above,
these are further converted, optionally without prior isolation with the elimination of water, to 1-alkyl-/1-aryl-substituted pyrazoles of the formula (V) 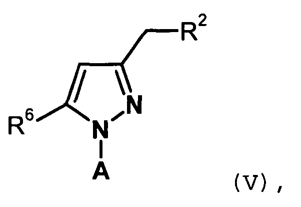 in which R², R⁶ and A have the meanings given above, these compounds of the general formula (V)
are converted to pyrazolecarboxylic acid derivatives of the formula (I), 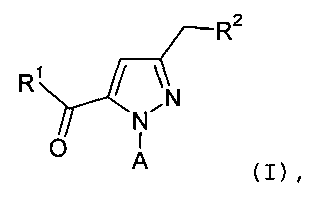 in which R¹, R² and A have the meanings given above.

2. Process for the preparation of compounds of the formula (I) according to Claim 1, where
R¹ is hydroxy, halogen, (C₁-C₆) alkoxy,
R² is hydroxy, halogen, O-(C=O) (C₁-C₆ alkyl, OSO₂ (C₁-C₆) alkyl, OSO₂ halo (C₁-C₆) alkyl,
A is (C₁-C₄) alkyl or
is the group 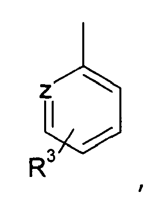
R³ is halogen, CN, NO₂, (C₁-C₆)-alkyl, halo (C₁-C₆)-alkyl, (C₁-C₆)alkoxy, halo(C₁-C₆)alkoxy,
Z is N.

3. Process for the preparation of compounds of the formula (I) according to either of Claims 1 and 2, where
R¹ is hydroxy, halogen, (C₁-C₄)alkoxy,
R² is hydroxy, halogen, O-(C=O)CH₃,
A is the group 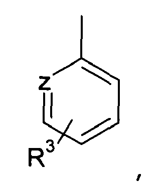
R³ is chlorine,
Z is N.

4. Process for the preparation of compounds of the formula (I) according to one of Claims 1 to 3, **characterized in that** n is 0.

5. Process for the preparation of compounds of the formula (I) according to one of Claims 1 to 4, **characterized in that** compounds of formula (II) 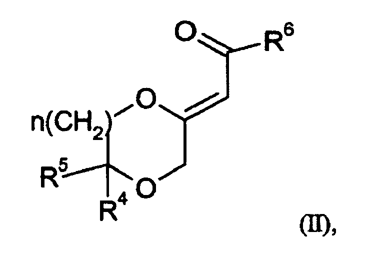 in which n is 0 and R⁴, R⁵ and R⁶ have the definitions indicated above,
are reacted first with nucleophiles of the formula (VI)
H₂L (VI), in which L is O, NH or NR⁷,
R⁷ is alkyl,
to give aminohydroxyoxopentenoates or hydroxy-2,4-dioxopentanoates of the formula (VII), which may be present in the form of two tautomeric forms (VIIa) and (VIIb) and may form a ring of the formula (VIIc), (VIId), 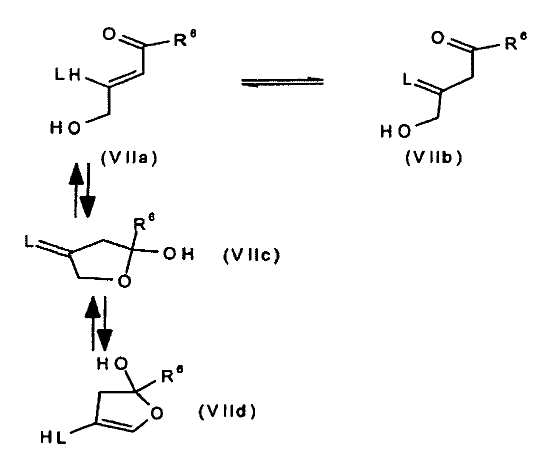 which are subsequently reacted with arylhydrazines of the formula (III)
A-NHIVH₂ (III),
in which A has the definitions indicated above,
to give 1-alkyl-/1-aryl-substituted dihydro-1H-pyrazoles of the formula (IV), 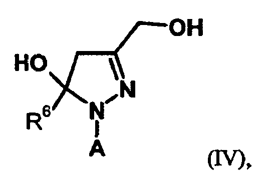 in which A and R⁶ have the definitions indicated above.

6. Process for the preparation of compounds of the formula (I) according to one of Claims 1 to 5, **characterized in that** process step (1) is carried out within a temperature range from 20°C to +100°C.

7. Process for the preparation of compounds of the formula (I) according to one of Claims 1 to 4, **characterized in that** 1 mol of a compound of the formula (II) is reacted with 0.8 mol to 2 mol of an alkyl- or arylhydrazine of the formula (III).

8. Process for the preparation of compounds of the formula (I) according to one of Claims 1 to 7, **characterized in that**
the compounds of the formula (IV) are converted, with the addition of a base, directly into the compounds of the formula (I), where
R¹ and R² are hydroxy, R⁶ is (C=O)Oalkyl, and
R³, R⁴, R⁵, A and Z have the meaning as in Claim 1.

9. Process for the preparation of compounds of the formula (I) according to Claim 8, **characterized in that** the base is selected from the group consisting of LiOH, NaOH, KOH and CsOH, and alcohols or water are used as solvents.

10. Process for the preparation of compounds of the formula (I) according to one of Claims 1 to 7, **characterized in that** process step (3) is carried out within a temperature range from 20°C to +150°C.

11. Process for the preparation of compounds of the formula (I) according to one of Claims 1 to 7 or 10, **characterized in that** process step (3) is carried out under acidic conditions with mineral or organic acids or under basic conditions with organic or inorganic bases.

12. Process for the preparation of compounds of the formula (I) according to one of Claims 1 to 7 or 10, **characterized in that** process step (3) is carried out with alcohol or combinations of alcohol/HCl, alcohol/FeCl₃, alcohol/H₂SO₄ or alcohol/alcoholate.

13. Process for the preparation of compounds of the formula (I) according to one of Claims 1 to 7, **characterized in that**
the compounds of the formula (IV) are converted, with the addition of an acid (HCl), directly into the compounds of the formula (I), where
R¹ is (C=O)Oalkyl, R² is hydroxy and R⁶ is (C=O) Oalkyl, and
R³, R⁴, R⁵, A and Z have the meaning as in Claim 1.

14. Compounds of the formula (IV) 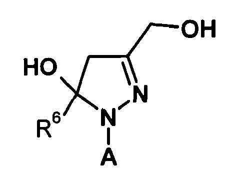 in which R⁶ is (C=O)Oalkyl and A has the meaning given in Claim 1.

15. Compounds of the general formula (VII) which can be present in the form of two tautomeric forms (VIIa) and (VIIb) and can form a ring of the formula (VIIc), (VIId), 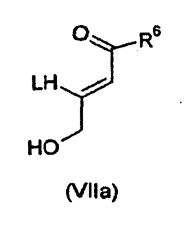 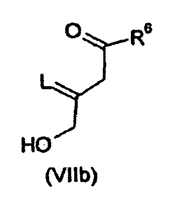 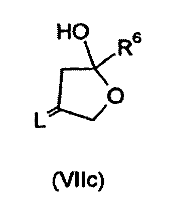 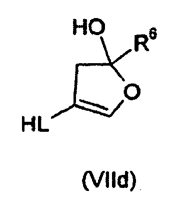 where L is O or NH and R⁶ is CF₃ or (C=O)Oalkyl.

## Revendications

1. Procédé pour la préparation de dérivés d'acide 5-pyrazolecarboxylique 1-alkyle-/1-aryle-substitués de formule générale (I) 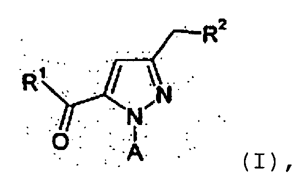 dans laquelle
R¹ représente le groupe hydroxy, un atome d'halogène, un groupe alcoxy, aryloxy,
R² représente le groupe hydroxy, un atome d'halogène, un groupe alcoxy, aryloxy, O-(C=O)alkyle, O-(C=O)O-alkyle, O(C=O)halogénoalkyle, OSO₂-alkyle, OSO₂-halogénoalkyle, OSO₂-aryle,
A représente un groupe alkyle ou représente le groupe 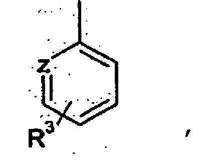
R³ représente un atome d'halogène, CN, NO₂, un groupe alkyle, cycloalkyle, halogénoalkyle, halogénocycloalkyle, alcoxy, halogénoalcoxy, alkylamino, dialkylamino, cycloalkylamino,
Z représente CH, N,
**caractérisé en ce qu'**on fait réagir des 1,4-dioxanes et 1,3-dioxolanes substitués de formule (II) 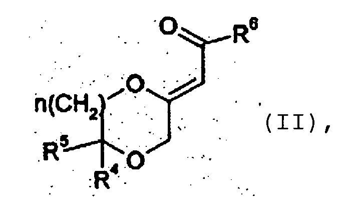 dans laquelle
R⁴, R⁵ représentent, indépendamment l'un de l'autre, un atome d'hydrogène, un groupe alkyle, aryle, arylalkyle, alcoxy,
R⁴, R⁵ en outre peuvent former un cycle saturé à 4, 5 ou 6 chaînons, éventuellement substitué, qui peut comporter 1-2 hétéroatomes choisis dans la série N, S, O,
R⁶ représente un groupe trihalogénométhyle, (C=O)O-alkyle, (C=O)O-halogénoalkyle,
n représente 0 ou 1,
avec des alkyl- ou arylhydrazines de formule (III)
^{A-}NHNH₂ (III),
dans laquelle A représente un groupe alkyle ou le groupe 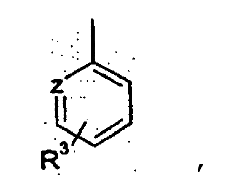
R³ représente un atome d'halogène, CN, NO₂, un groupe alkyle, cycloalkyle, halogénoalkyle, halogénocycloalkyle, alcoxy, halogénoalcoxy, alkylamino, dialkylamino, cycloalkylamino,
Z représente CH, N,
pour aboutir à des dihydro-1H-pyrazoles 1-alkyle-/ 1-aryle substitués de formule (IV), 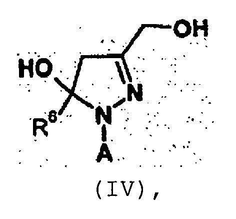 dans laquelle R⁶, A ont les significations indiquées plus haut,
éventuellement on convertit encore ces derniers, sans isolement préalable, avec élimination d'eau, en pyrazoles 1-alkyle-/1-aryle-substitués de formule (V) 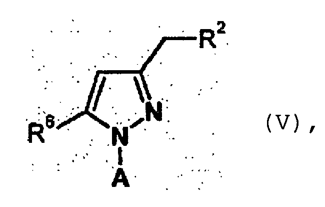 dans laquelle R², R⁶ et A ont les significations indiquées plus haut,
on convertit ces composés de formule générale (V),
en dérivés d'acide pyrazolecarboxylique de formule (I), 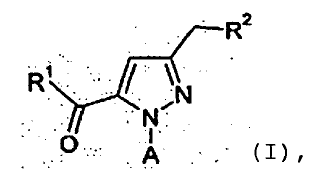 dans laquelle R¹, R² et A ont les significations indiquées plus haut.

2. Procédé pour la préparation de composés de formule (I) selon la revendication 1, dans lequel
R¹ représente le groupe hydroxy, un atome d'halogène, un groupe alcoxy en C₁-C₆,
R² représente le groupe hydroxy, un atome d'halogène, un groupe O-(C=O) alkyle (C₁-C₆), OSO₂-alkyle(C₁-C₆), OSO₂-halogénoalkyle (C₁-C₆),
A représente un groupe alkyle en C₁-C₄ ou représente le groupe 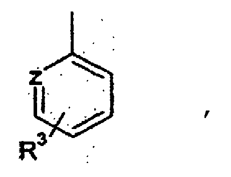
R³ représente un atome d'halogène, CN, NO₂, un groupe alkyle en C₁-C₆, halogénoalkyle(C₁-C₆), alcoxy (C₁-C₆) halogénoalcoxy(C₁-C₆),
Z représente N.

3. Procédé pour la préparation de composés de formule (I) selon l'une quelconque des revendications 1 et 2, dans lequel
R¹ représente le groupe hydroxy, un atome d'halogène, un groupe alcoxy en C₁-C₄,
R² représente le groupe hydroxy, un atome d' halogène, le groupe O-(C=O)CH₃,
A représente le groupe 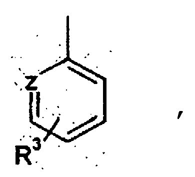
R³ représente un atome de chlore,
Z représente N.

4. Procédé pour la préparation de composés de formule (I) selon l'une quelconque des revendications 1 à 3, **caractérisé en ce que** n représente 0.

5. Procédé pour la préparation de composés de formule (I) selon l'une quelconque des revendications 1 à 4, **caractérisé en ce qu'**on fait réagir des composés de formule (II) 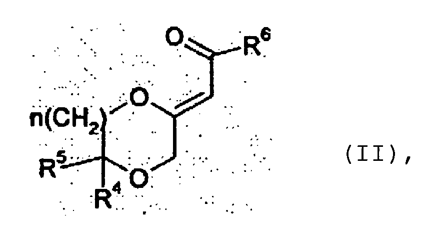 dans laquelle n représente 0 et R⁴, R⁵ et R⁶ ont les significations indiquées plus haut,
d'abord avec des composés nucléophiles de formule (VI)
H₂L (VI), dans laquelle L représente O, NH ou NR⁷,
R⁷ représente un groupe alkyle,
pour aboutir à des aminohydroxyoxopenténoates ou des hydroxy-2,4-dioxopentanoates de formule (VII), qui peuvent être présents sous forme de deux formes tautomères (VIIa) et (VIIb) et peuvent former un cycle de formule (VIIc), (VIId), 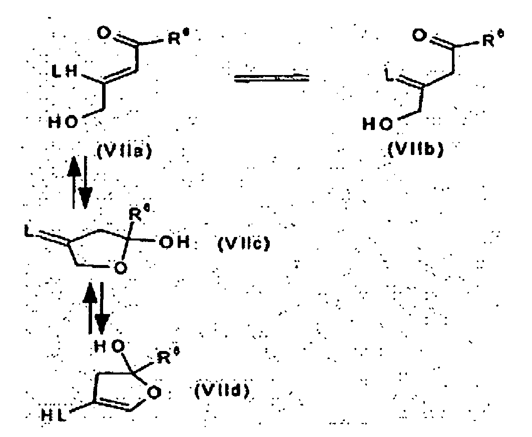 et on fait ensuite réagir ces derniers avec des arylhydrazines de formule (III)
A-NHNH₂ (III),
dans laquelle A a les significations indiquées plus haut,
pour aboutir à des dihydro-1H-pyrazoles 1-alkyle-/ 1-aryle substitués de formule (IV) 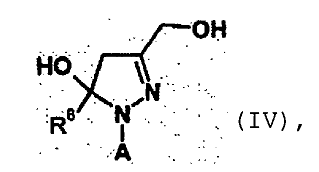 dans laquelle A et R⁶ ont les significations indiquées plus haut.

6. Procédé pour la préparation de composés de formule (I) selon l'une quelconque des revendications 1 à 6, **caractérisé en ce que** l'exécution de l'étape (1) du procédé s'effectue dans une plage de température de -20 °C à +100 °C.

7. Procédé pour la préparation de composés de formule (I) selon l'une quelconque des revendications 1 à 4, **caractérisé en ce qu'**on fait réagir 1 mole d'un composé de formule (II) avec 0,8 mole à 2 moles d'une alkyl- ou arylhydrazine de formule (III).

8. Procédé pour la préparation de composés de formule (I) selon l'une quelconque des revendications 1 à 7, **caractérisé en ce**
**qu'**on convertit les composés de formule (IV), avec addition d'une base, directement en les composés de formule (I),
R¹ et R² représentant le groupe hydroxy, R⁶ représentant un groupe (C=O)O-alkyle, et
R³, R⁴, R⁵, A et Z ayant la signification selon la revendication 1.

9. Procédé pour la préparation de composés de formule (I) selon la revendication 8, **caractérisé en ce que** la base est choisie dans le groupe constitué par LiOH, NaOH, KOH et CsOH et on utilise comme solvant des alcools ou l'eau.

10. Procédé pour la préparation de composés de formule (I) selon l'une quelconque des revendications 1 à 7, **caractérisé en ce que** l'exécution de l'étape (3) du procédé a lieu dans une plage de température de 20 °C à +150 °C.

11. Procédé pour la préparation de composés de formule (I) selon l'une quelconque des revendications 1 à 7 ou 10, **caractérisé en ce que** l'exécution de l'étape (3) du procédé a lieu dans des conditions acides avec des acides minéraux ou organiques ou dans des conditions basiques avec des bases organiques ou inorganiques.

12. Procédé pour la préparation de composés de formule (I) selon l'une quelconque des revendications 1 à 7, **caractérisé en ce que** l'exécution de l'étape (3) du procédé a lieu avec un alcool ou des associations d'alcool/HCl, alcool/FeCl₃, alcool/H₂SO₄ ou alcool/ alcoolate.

13. Procédé pour la préparation de composés de formule (I) selon l'une quelconque des revendications 1 à 7, **caractérisé en ce**
**qu'**on convertit les composés de formule (IV), avec addition d'un acide (HCl), directement en les composés de formule (I),
R¹ représentant un groupe (C=O)O-alkyle, R² représentant le groupe hydroxy et R⁶ représentant un groupe (C=O)O-alkyle, et
R³, R⁴, R⁵, A et Z ayant la signification selon la revendication 1.

14. Composés de formule (IV) 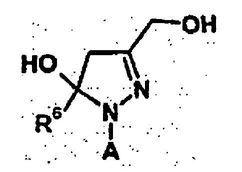 dans laquelle R⁶ représente un groupe (C=O)O-alkyle et A a la signification indiquée dans la revendication 1.

15. Composés de formule générale (VII), qui peuvent se trouver sous forme de deux formes tautomères (VIIa) et (VIIb) et peuvent former un cycle de formule (VIIc), (VIId), 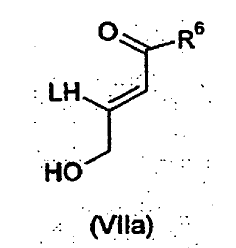 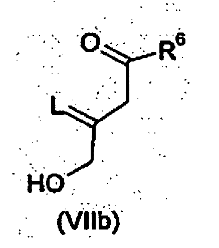 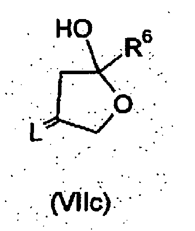 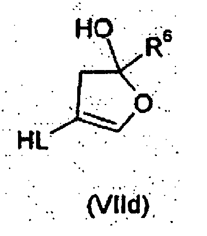 L représentant O ou NH et R⁶ représentant CF₃ ou (C=O)O-alkyle.
